(19) Europäisches Patentamt
European Patent Office
Office européen des brevets

(11) **EP 3 401 317 B1**

(12) # EUROPEAN PATENT SPECIFICATION

(45) Date of publication and mention of the grant of the patent:
**04.05.2022 Bulletin 2022/18**

(21) Application number: **16883378.8**

(22) Date of filing: **05.12.2016**

(51) International Patent Classification (IPC):
***C07D 495/04*** (2006.01) ***C07D 495/20*** (2006.01)
***C07D 495/10*** (2006.01) ***C07D 519/00*** (2006.01)
***C07F 7/10*** (2006.01) ***H01L 51/54*** (2006.01)
***H01L 51/00*** (2006.01) ***C08G 61/12*** (2006.01)

(52) Cooperative Patent Classification (CPC):
**C07D 495/04; C07D 495/10; C07D 495/14; C07D 495/20; C07D 519/00; C07F 7/10; C07F 9/6561; C09B 57/00; C09K 11/06; H01L 51/0071;** H01L 51/0085; H01L 51/5016; Y02E 10/549

(86) International application number:
**PCT/CN2016/108593**

(87) International publication number:
**WO 2017/118252 (13.07.2017 Gazette 2017/28)**

(54) **SULFONE-CONTAINING FUSED HETEROCYCLIC COMPOUNDS AND APPLICATIONS THEREOF**

SULFONHALTIGE KONDENSIERTE HETEROCYCLISCHE VERBINDUNGEN UND ANWENDUNGEN DAVON

COMPOSÉS HÉTÉROCYCLIQUES CONDENSÉS CONTENANT UNE FONCTION SULFONE ET LEURS APPLICATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2016 CN 201610013130**

(43) Date of publication of application:
**14.11.2018 Bulletin 2018/46**

(73) Proprietor: **Guangzhou Chinaray Optoelectronic Materials Ltd.**
**Guangzhou, Guangdong 510663 (CN)**

(72) Inventors:
- **PAN, Junyou**
  **Guangzhou**
  **Guangdong 510663 (CN)**
- **HE, Ruifeng**
  **Guangzhou**
  **Guangdong 510663 (CN)**
- **PENG, Feng**
  **Guangzhou**
  **Guangdong 510663 (CN)**
- **YANG, Wei**
  **Guangzhou**
  **Guangdong 510663 (CN)**

(74) Representative: **Hoffmann Eitle**
**Patent- und Rechtsanwälte PartmbB**
**Arabellastraße 30**
**81925 München (DE)**

(56) References cited:
**WO-A1-2016/021989** **CN-A- 103 718 316**
**CN-A- 104 263 351** **CN-A- 105 154 067**
**KR-A- 20110 113 469**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present disclosure relates to the field of organic electroluminescent materials, especially to a sulfone-containing fused heterocyclic compound, a polymer whose repeating unit comprising one sulfone-containing fused heterocyclic structural unit, a mixture and formulation comprising the sulfone-containing fused heterocyclic compound, and its application in organic electronic devices.

### BACKGROUND

With the characteristics of structural diversity, relatively low manufacturing cost, superior photoelectric property, etc., organic semiconductor materials show great potential for a use in optoelectronic devices such as organic light-emitting diode (OLED), such as flat panel displays and lighting.

In order to improve the luminescence properties of the organic light-emitting diodes and promote the large-scale industrialization of the organic light-emitting diodes, a variety of material systems with organic photoelectric properties have been widely developed. However, the properties of OLED, especially its lifetime, still needs to be improved. For phosphorescent OLED, for example, the lifetime of the device is determined by the stability of its host material. For another example, a new generation of OLED material, namely thermally activated delayed fluorescent material (TADF), has a high efficiency, but low lifetime mainly due to the lack of a suitable host material. Still for another example, during the printing of OLED, there is an urgent need for materials with good properties, and at the same time good solubility, film-forming property, and thermal stability, especially for the host materials. Therefore, new high-property host materials urgently need to be developed. CN 103 718 316 is related to compounds for use in electronic devices, such as organic electroluminescent devices. KR 2011 0113469 relates to a heterocycic compound and an organic electroluminescent device comprising the same as a light emitting material.

Wherein, sulfone-containing fused heterocyclic photoelectric materials have been widely used in optoelectronic devices due to their good carrier transport property and photoelectric properties. However, there are still some limitations on the carrier balance and stability for the current reported sulfone-containing fused heterocyclic materials.

### SUMMARY

Based on this, it is necessary to provide a sulfone-containing fused heterocyclic compound with good carrier balance ability and high stability.

According to one aspect of the present disclosure, a sulfone-containing fused heterocyclic compound with the following general formula (1) is provided:

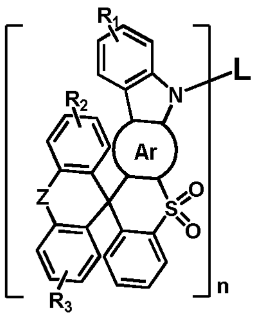

(1);

wherein L represents an aromatic ring with a carbon atom number of 6 to 60, or a heteroaromatic ring with a carbon atom number of 3 to 60;

-Z- represents a single bond, -N(R)-, $-C(R)_2-$, $-Si(R)_2-$, -O-, -C=N(R)-, $-C=C(R)_2-$, -P(R)-, -P(=O)R-,-S-,-(S=O)-or $-SO_2-$;

$R_1$, $R_2$, $R_3$ and R each independently represents H, D, F, CN, aralkyl, alkenyl, alkynyl, nitrile, amine, nitro, acyl, alkoxy, carbonyl, sulfonyl, hydroxyl, alkyl with a carbon atom number of 1 to 30, cycloalkyl with a carbon atom number of 3 to 30, aromatic hydrocarbyl with a carbon atom number of 6 to 60, or aromatic heterocyclyl with a carbon atom number of 3 to 60.;

Ar represents an aromatic ring with a carbon atom number of 6 to 40, or a heteroaromatic ring with a carbon atom

number of 3 to 40;
n is selected from 2, 3, 4, 5, or 6.

According to another aspect of the present disclosure, there is provided a polymer whose repeating unit comprises any one of the above-described sulfone-containing fused heterocyclic compounds.
According to further aspect of the present disclosure, there is provided a mixture comprising any one of the above-described sulfone-containing fused heterocyclic compounds and organic functional material, or the mixture comprises any one of the above-described polymers and organic functional material.
The organic functional material is at least one selected from the group consisting of a hole injection material, a hole transport material, an electron transport material, an electron injection material, an electron blocking material, a hole blocking material, a light-emitting material, a host material, and an organic dye.
According to yet another aspect of the present disclosure, there is provided a formulation comprising any one of the above-described sulfone-containing fused heterocyclic compounds and at least one organic solvent;

or, the formulation comprises any one of the above-described polymers and at least one organic solvent;
or, the formulation comprises any one of the above-described mixtures and at least one organic solvent.

According to still another aspect of the present disclosure, there is provided an application of any one of the above-described sulfone-containing fused heterocyclic compounds or any one of the above-described polymers in electronic devices.
According to another aspect of the present disclosure, there is provided an organic electronic device comprising any one of the above-described sulfone-containing fused heterocyclic compounds, any one of the above-described polymers or any one of the above-described mixtures.
The use of the above sulfone-containing fused heterocyclic compound in OLED, particularly as a host material in light-emitting layer, can provide higher quantum efficiency and enhance the light-emitting stability and extend the device lifetime. The possible reasons are as follows, but not limited thereto, the sulfone-containing fused heterocyclic compounds have good electron and hole bipolar transport properties, and can effectively balance carrier transport, and improve the efficiency of carrier to generate excitons, leading to good structural stability for the sulfone-containing fused heterocyclic compounds, which provides the possibility to improve the photoelectric properties and device stability for related devices.

## BRIEF DESCRIPTION OF THE DRAWINGS

FIG.1 is a LUMO electron cloud distribution diagram of the sulfone-containing fused heterocyclic compound prepared in Example 1.
FIG.2 is a HOMO electron cloud distribution diagram of the sulfone-containing fused heterocyclic compound prepared in Example 1.
FIG.3 is a LUMO electron cloud distribution diagram of the sulfone-containing fused heterocyclic compound prepared in Example 2.
FIG.4 is a HOMO electron cloud distribution diagram of the sulfone-containing fused heterocyclic compound prepared in Example 2.

## DETAILED DESCRIPTION OF THE EMBODIMENTS

In order to make the purpose, technical solution and effects of the present disclosure clearer and more specific, the present disclosure will be furthermore described in detail below. It should be noted that, the specific embodiment illustrated herein is merely for the purpose of explanation, and should not be deemed to limit the disclosure.In the context, formulation and printing ink, or ink, have the same meaning and they can be used interchangeably. In the context, host material, matrix material, Host or Matrix material has the same meaning and they can be used interchangeably. In the context, metal organic complex, and organometallic complex have the same meaning and can be used interchangeably.
A sulfone-containing fused heterocyclic compound of an embodiment with the following general formula (1):

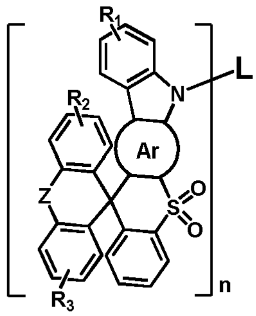

(1);

wherein L represents an aromatic ring with a carbon atom number of 6 to 60, or a heteroaromatic ring with a carbon atom number of 3 to 60.

-Z- represents a single bond, -N(R)-, $-C(R)_2-$, $-Si(R)_2-$, -O-, -C=N(R)-, $-C=C(R)_2-$ , -P(R)-, -P(=O)R-, -S-, -(S=O)- or $-SO_2-$.

In one embodiment, when -Z- represents a single bond, it means that one carbon atom on the benzene ring substituted with $R_2$ and one carbon atom on the benzene ring substituted with $R_3$ are linked by a single bond.

In one embodiment, -Z- may also be absent, i.e., which means that there is no bond linking between the benzene ring substituted with $R_2$ and the benzene ring substituted with $R_3$ on the position indicated by -Z-.

Specifically, R in brackets of -N(R)-, $-C(R)_2-$, $-Si(R)_2-$, -C=N(R)-, $-C=C(R)_2-$, -P(R) - and -P(=O)R- represents a substituent on the corresponding atom or group.

$R_1$, $R_2$, $R_3$ and R independently represent H, D, F, CN, aralkyl, alkenyl, alkynyl, nitrile, amine, nitro, acyl, alkoxy, carbonyl, sulfonyl, hydroxyl, alkyl with a carbon atom number of 1 to 30, cycloalkyl with a carbon atom number of 3 to 30, aromatic hydrocarbyl with a carbon atom number of 6 to 60, or aromatic heterocyclyl with a carbon atom number of 3 to 60, respectively.

Ar represents an aromatic ring with a carbon atom number of 6 to 40, or a heteroaromatic ring with a carbon atom number of 3 to 40

n is selected from 2, 3, 4, 5, or 6.

In certain embodiments, Ar as shown in general formula (1) may be selected from an unsubstituted or substituted aromatic ring or heteroaromatic ring with 2 to 30 carbon atoms. In some embodiments, Ar may be selected from unsubstituted or substituted aromatic ring or heteroaromatic ring with 2 to 20 carbon atoms. In some embodiments, Ar may be selected from unsubstituted or substituted aromatic ring or heteroaromatic ring with 2 to 15 carbon atoms.

In some embodiments, for Ar, the aromatic ring system comprises 5 to 15 carbon atoms in the ring system, such as 5 to 10 carbon atoms. The heteroaromatic ring system comprises 2 to 15 carbon atoms in the ring system, such as 2 to 10 carbon atoms, and at least one heteroatom, provided that the total number of carbon atoms and heteroatoms is at least 4. The heteroatom may be selected from Si, N, P, O, S, and/or Ge, and in some embodiments, the heteroatom may be selected from Si, N, P, O, and/or S.

The aromatic ring system or aromatic group refers to hydrocarbyl comprising at least one aromatic ring, including monocyclic group and polycyclic ring system. The heteroaromatic ring system or heteroaromatic group refers to hydrocarbyl (containing heteroatoms) comprising at least one heteroaromatic ring, including monocyclic group and polycyclic ring system. Such polycyclic rings may have two or more rings, wherein two carbon atoms are shared by two adjacent rings, i.e., fused ring. At least one of such polycyclic rings is aromatic or heteroaromatic. For the purpose of the present dislosure, the aromatic or heteroaromatic ring systems not only include aromatic or heteroaromatic systems, but also a plurality of aryl or heteroaryl groups in the systems may be interrupted by short non-aromatic units (<10% of non-H atoms, preferably less than 5% of non-H atoms, such as C, N or O atoms). Therefore, systems such as 9,9'-spirobifluorene, 9,9-diarylfluorene, triarylamine, diaryl ether and the like are also considered to be aromatic ring systems for the purpose of this disclosure.

Specifically, examples of the aromatic group include: benzene, naphthalene, anthracene, phenanthrene, perylene, tetracene, pyrene, benzopyrene, triphenylene, acenaphthene, fluorene, and derivatives thereof.

Specifically, examples of the heteroaromatic group include: furan, benzofuran, thiophene, benzothiophene, pyrrole, pyrazole, triazole, imidazole, oxazole, oxadiazole, thiazole, tetrazole, indole, carbazole, pyrroloimidazole, pyrrolopyrrole, thienopyrrole, thienothiophene, furopyrrole, furofuran, thienofuran, benzisoxazole, benzisothiazole, benzimidazole, pyridine, pyrazine, pyridazine, pyrimidine, triazine, quinoline, isoquinoline, phthalazine, cinnoline, quinoxaline, phenanthridine, perimidine, quinazoline, quinazolinone and derivatives thereof.

Specifically, Ar as shown in general formula (1) may be one structural group selected from the group consisting of:

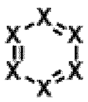 、 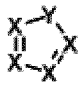 、 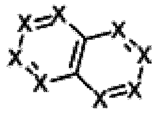 、 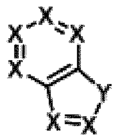 and 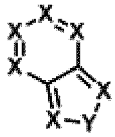 ;

wherein X is $CR^1$ or N;

Y is selected from the group consisting of $CR^2R^3$, $SiR^4R^5$, $NR^6$, C(=O), S and O;
$R^1$, $R^2$, $R^3$, $R^4$, $R^5$, and $R^6$ each independently represents H, or D(deuterium), or linear alkyl groups containing 1 to 20 carbon atoms, linear alkoxy groups containing 1 to 20 carbon atoms or linear thioalkoxy groups containing 1 to 20 carbon atoms, or branched or cyclic alkyl groups containing 3 to 20 carbon atoms, branched or cyclic alkoxy groups containing 3 to 20 carbon atoms or branched or cyclic thioalkoxy groups containing 3 to 20 carbon atoms or branched or cyclic silyl groups containing 3 to 20 carbon atoms, or substituted keto groups containing 1 to 20 carbon atoms, alkoxycarbonyl groups containing 2 to 20 carbon atoms, aryloxycarbonyl groups containing 7 to 20 carbon atoms, cyano group (-CN), carbamoyl group ($-C(=O)NH_2$), haloformyl group (-C(=O)-A, wherein A represents halogen atom), formyl group (-C(=O)-H), isocyano group, isocyanate group, thiocyanate group, or isothiocyanate group, hydroxyl group, nitro group, $CF_3$ group, Cl, Br, F, or substituted or unsubstituted aromatic or heteroaromatic ring systems containing 5 to 40 ring atoms, aryloxy or heteroaryloxy groups containing 5 to 40 ring atoms, or combination of these systems, wherein one or more groups $R^1$, $R^2$, $R^3$, $R^4$, $R^5$, and $R^6$ may form monocyclic or polycyclic aliphatic or aromatic ring systems with each other and/or with a ring bonded thereto.

Further, Ar may be selected from one of the following structural groups:

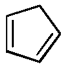, 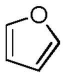, 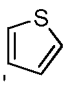, 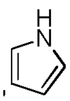, 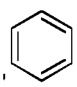, 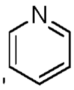, 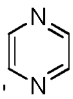, 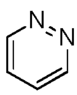, 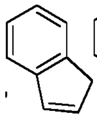, 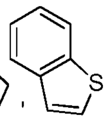, 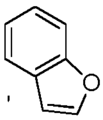, 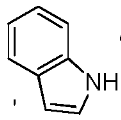, 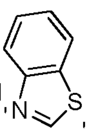, 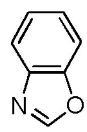
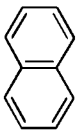, 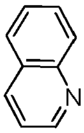, 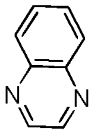, 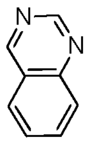, 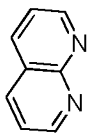, 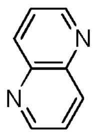, 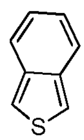, 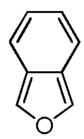, 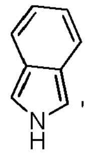, 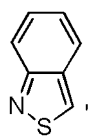, 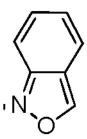 and 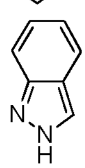

Specifically, for the above sulfone-containing fused heterocyclic compound, the sulfone-containing fused heterocyclic compound as shown in general formula (1) may be further represented by any one of the following chemical formulas (2) to (6) :

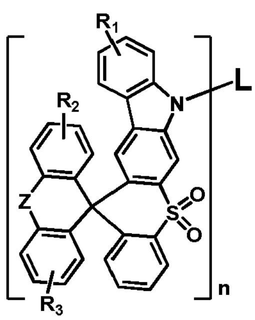 (2), 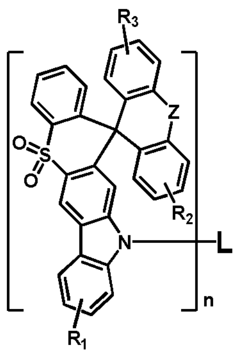 (3), 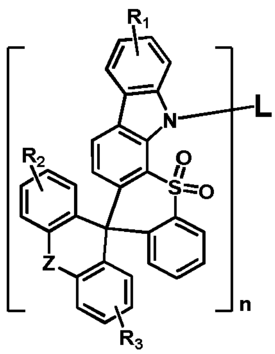 (4),

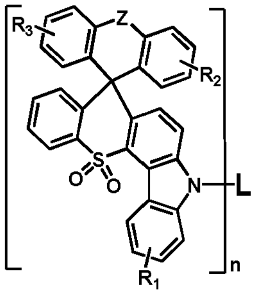 (5), 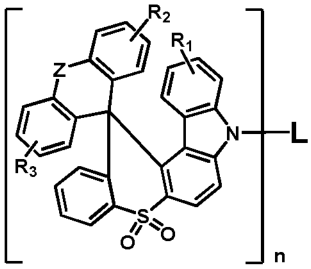 (6).

Specifically, the definitions of Z, L, $R_1$, $R_2$, $R_3$, R in formulas (2) to (6) and Ar can be seen in the description in general formula (1).

In other embodiments, $R_1$, $R_2$, and $R_3$ as shown in general formula (1) each independently represents alkyl with a carbon atom number of 1 to 18 or cycloalkyl with a carbon atom number of 3 to 18 or aromatic group with a carbon atom number of 6 to 18 or heteroaromatic group with a carbon atom number of 3 to 18. In certain embodiments, $R_1$, $R_2$, and $R_3$ each independently represents alkyl with a carbon atom number of 1 to 10 or cycloalkyl with a carbon atom number of 3 to 10 or aromatic group with a carbon atom number of 6 to 15 or heteroaromatic group with a carbon atom number of 3 to 15.

In certain embodiments, L as shown in general formula (1) is an aromatic group with a carbon atom number of 6 to 40, or a heteroaromatic group with a carbon atom number of 3 to 40. In certian embodiments, L is an aromatic group with a carbon atom number of 6 to 30, or a heteroaromatic group with a carbon atom number of 3 to 30. In certian embodiments, L is an aromatic group with a carbon atom number of 6 to 20, or a heteroaromatic group with a carbon atom number of 3 to 20

Suitable examples of heteroaromatic group that can be used as L include, but not limited to, groups such as benzene, naphthalene, anthracene, phenanthrene, pyrene, pyridine, pyrimidine, triazine, fluorene, dibenzothiophene, silafluorene, carbazole, thiophene, furan, thiazole, triphenylamine, triphenylphosphine oxide, tetraphenyl silicane, spirofluorene, spirosilabifluorene and the like, preferably groups such as a single bond, benzene, pyridine, pyrimidine, triazine, carbazole, and the like.

In one of the embodiments, suitable examples that can be used as L include groups such as methyl, benzene, naphthalene, anthracene, phenanthrene, pyrene, pyridine, pyrimidine, triazine, fluorene, dibenzothiophene, silafluorene, carbazole, thiophene, furan, thiazole, triphenylamine, triphenylphosphine oxide, tetraphenyl silicane, spirofluorene, spirosilabifluorene and the like, preferably groups such as benzene, pyridine, pyrimidine, triazine, carbazole and the like.

In one embodiment, in general formula (1), L may be selected from one of the following structural groups:

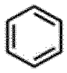 、 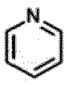 、 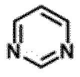 、 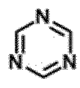 、 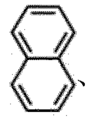 、 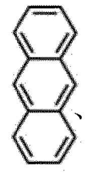 、 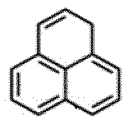 、 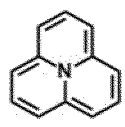 、 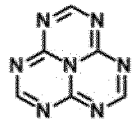 、

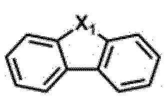 、 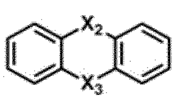 、 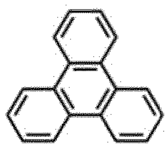 、 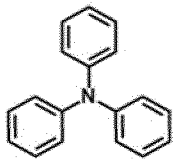 、 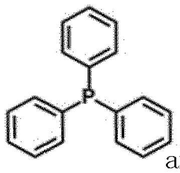 and 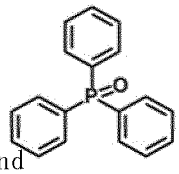 ;

wherein $X_4$, $X_5$ and $X_6$ each independently represents N(R), $C(R)_2$, $Si(R)_2$, O, C=N(R), $C=C(R)_2$, P(R), P(=O)R, S, S=O, $SO_2$ or absent, wherein at least one of $X_2$ and $X_3$ is not absent. R represents H, D, F, CN, aralkyl, alkenyl, alkynyl, nitrile, amine, nitro, acyl, alkoxy, carbonyl, sulfonyl, hydroxyl, alkyl with a carbon atom number of 1 to 30, cycloalkyl with a carbon atom number of 3 to 30, aromatic hydrocarbyl with a carbon atom number of 6 to 60, or aromatic heterocyclyl with a carbon atom number of 3 to 60, respectively.

In one embodiment, suitable Z, $X_4$, $X_5$, $X_6$ may independently be selected from the group consisting of N(R), $C(R)_2$, $Si(R)_2$, O, and S. In certain embodiments, Z may be a single bond.

In the general formula (1), n is an integer of 2 to 6, further, n is an integer of 2 to 5, still further, n is an integer of 2 to 4, still further, n is an integer of 2 to 3, still further, n is 2.

Specifically, the sulfone-containing fused heterocyclic compound according to the present disclosure is selected

from one of the following structural formulas:

| | | |
|---|---|---|
| 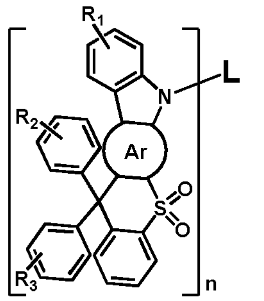 | 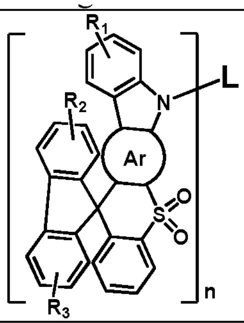 | 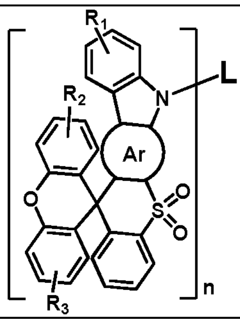 |
| 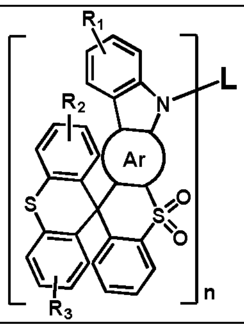 | 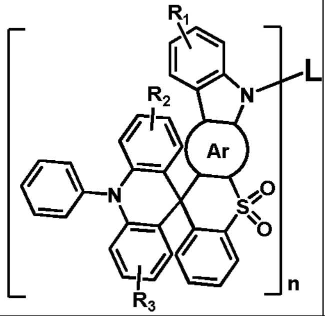 | 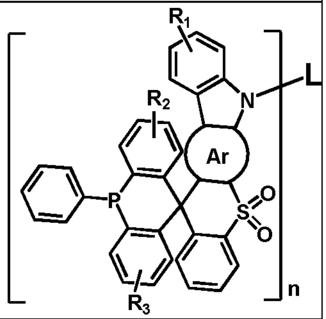 |
| 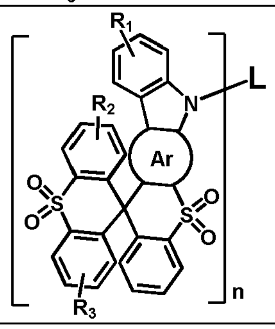 | 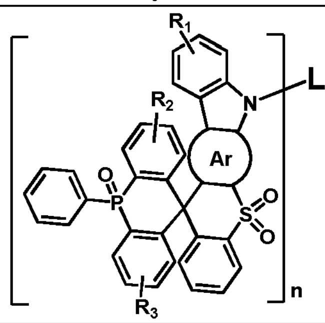 | 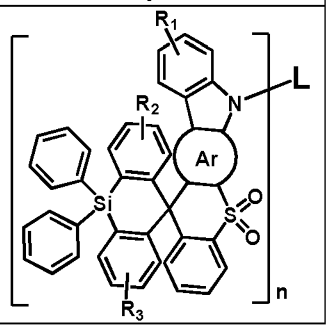 |

| | | |
|---|---|---|
| 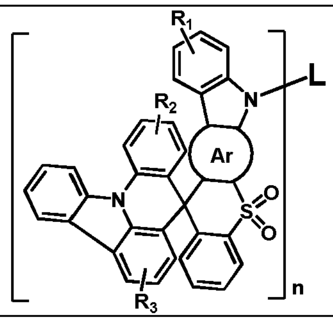 | 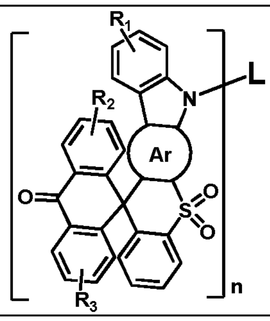 | 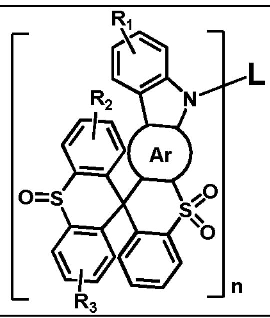 |

wherein, Ar, L, $R_1$, $R_2$, $R_3$ and n are as defined above.

In one embodiment, the above sulfone-containing fused heterocyclic compound has thermally activated delayed fluorescent TADF characteristic. According to the principle of thermally activated delayed fluorescent TADF material (see Adachi et al., Nature, Vol 492, 234, (2012)), when the difference between the singlet and triplet energy levels of the organic compound $\Delta E$ ($S_1$-$T_1$) is small enough, the triplet excitons of the organic compound can be converted to singlet excitons by reverse internal conversion, thereby achieving efficient light emission. In general, TADF material is obtained by linking an electron donor to an electron deficiency or electron acceptor group, that is, having a distinct D-A structure. That is, by the way of linking an electron donor to an electron deficiency or electron acceptor group, it results in the complete separation of the electron cloud distributions of highest occupied molecular orbital (HOMO) and lowest unoccupied molecular orbital (LUMO), and the reduction of the energy difference of the organic compound between singlet state($S_1$) and triplet state($T_1$) $\Delta E$ ($S_1$-$T_1$) , but it has an adverse effect on light-emitting efficiency and stability.

In one embodiment, the sulfone-containing fused heterocyclic compound according to the present disclosure

has smaller $\Delta E(S_1-T_1)$, and generally $\Delta E(S_1-T_1) \leq 0.25$ eV, $\Delta E(S_1-T_1) \leq 0.20$ eV in other embodiments, $\Delta E(S_1-T_1) \leq 0.15$ eV in other embodiments, $\Delta E(S_1-T_1) \leq 0.12$ eV in other embodiments, $\Delta E(S_1-T_1) \leq 0.09$ eV in other embodiments.

In another embodiment, in the compound according to the present disclosure, the electron cloud distributions of highest occupied molecular orbital (HOMO) and lowest unoccupied molecular orbital (LUMO) are not completely separated, at least partially overlapped, and preferably most overlapped. The energy levels, electron cloud distributions and singlet ($S_1$) and triplet ($T_1$) energy levels of HOMO and LUMO described above can be obtained by quantum chemical simulations, and specific methods are described below.

The sulfone-containing fused heterocyclic compound according to the present disclosure can be used as functional material in electronic devices. Organic functional materials can be classified into a hole injection material (HIM), a hole transport material (HTM), an electron transport material (ETM), an electron injection material (EIM), an electron blocking material (EBM), a hole blocking material (HBM), emitter, host material, and an organic dye. Specifically, the compound according to the present disclosure can be used as a host material, or an electron transport material or a hole transport material. Further, the compound according to the present disclosure can be used as a phosphorescent host material.

Generally, phosphorescent host materials must have a proper triplet energy level, i.e., $T_1$. In certain embodiments, the compound according to the present disclosure has $T_1 \geq 2.2$ eV, $T_1 \geq 2.4$ eV in other embodiments, $T_1 \geq 2.6$ eV in other embodiments, $T_1 \geq 2.65$ eV in other embodiments, $T_1 \geq 2.7$ eV in other embodiments.

Typically, the triplet energy level $T_1$ of the organic compound depends on the substructure having the largest conjugated system in the compound. Generally, $T_1$ decreases as the conjugated system increases. In certain embodiments, the substructure as shown in following general formula (1a) in general formula (1) has the largest conjugated system:

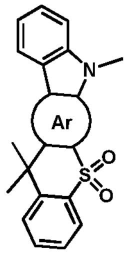

(1a);

In certain embodiments, in the case where the substituents are removed from general formula (1a), the number of ring atoms is no more than 30, no more than 26 in other embodiments, the number of ring atoms is no more than 22 in other embodiments, and the number of ring atoms is no more than 20 in other embodiments.

In other embodiments, in the case where the substituents are removed from general formula (1a), the organic compound has $T_1 \geq 2.2$ eV, $T_1 \geq 2.4$ eV in other embodiments, $T_1 \geq 2.6$ eV in other embodiments, $T_1 \geq 2.65$ eV in other embodiments, $T_1 \geq 2.75$ eV in other embodiments.

Generally, the sulfone-containing fused heterocyclic compound according to the present disclosure has a glass transition temperature $T_g \geq 100$ °C. In certain embodiments, $T_g \geq 120$ °C. In certain embodiments, $T_g \geq 140$°C. In certain embodiments, $T_g \geq 160$ °C. In certain embodiments, $T_g \geq 180$ °C. It is shown that the above sulfone-containing fused heterocyclic compounds have good thermal stability and can be used as phosphorescent host material.

In addition, a synthetic method related to the sulfone-containing fused heterocyclic compound is also provided. Generally, a fused heterocycle with S and carbonyl can be monobrominated first, and then the carbonyl on the fused heterocyclic with bromine can be attacked with Grignard reagent or lithium salt containing Z group, followed by ring closing by dehydration, and the brominated bromo group is made into a boric acid ester group, and then coupled with o-nitrohalobenzene, after ring closing with nitro, coupled with an L group, and finally S is oxidized, to obtain the target compound.

The following are non-limiting specific examples of the sulfone-containing fused heterocyclic compound.

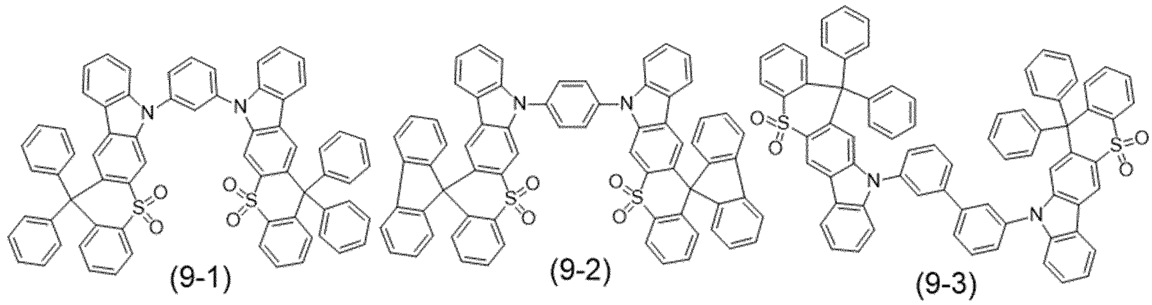

(9-1) (9-2) (9-3)

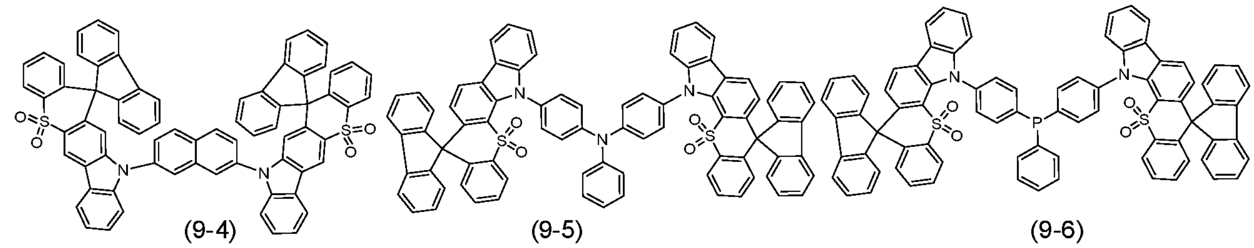

(9-4) (9-5) (9-6)

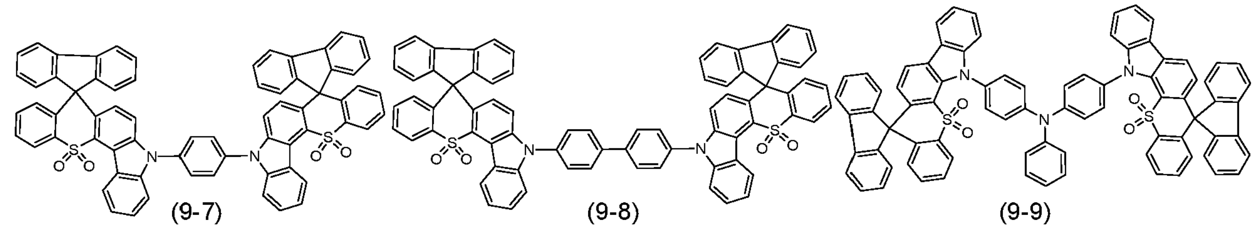

(9-7) (9-8) (9-9)

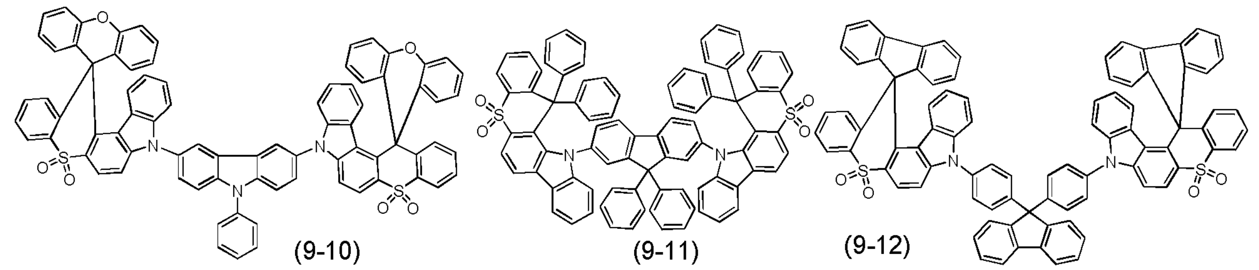

(9-10) (9-11) (9-12)

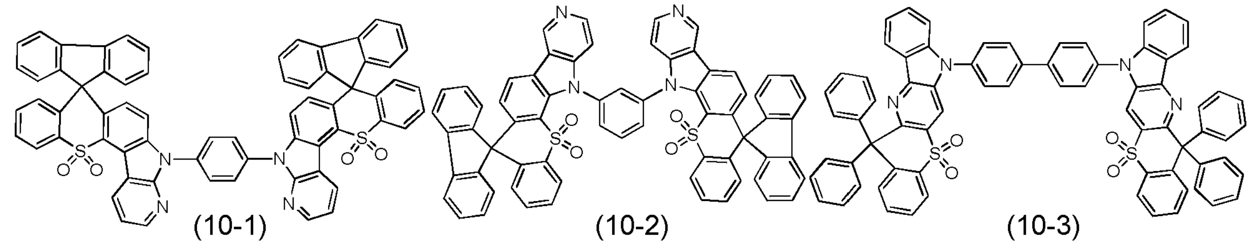

(10-1) (10-2) (10-3)

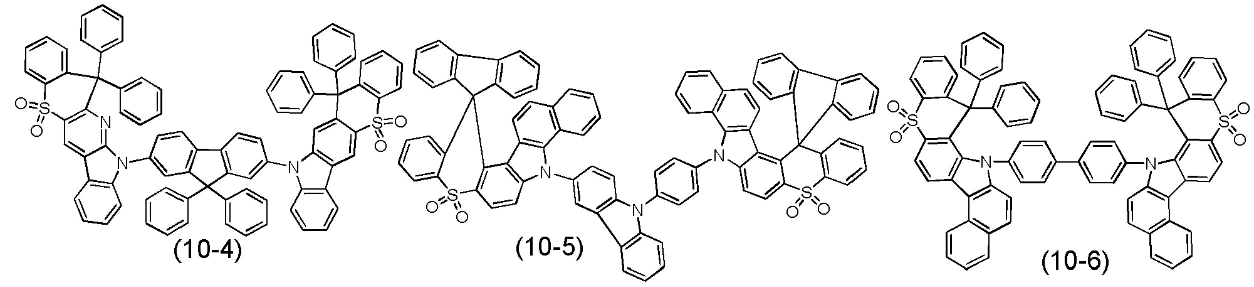

(10-4) (10-5) (10-6)

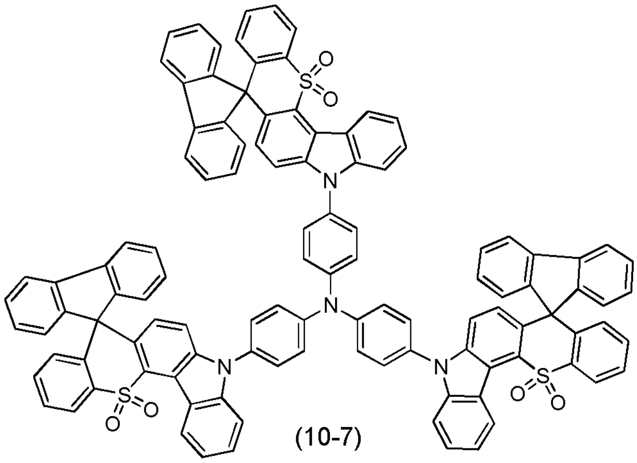

(10-7)

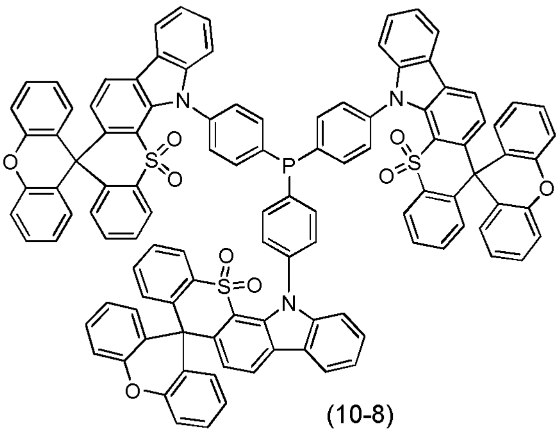

(10-8)

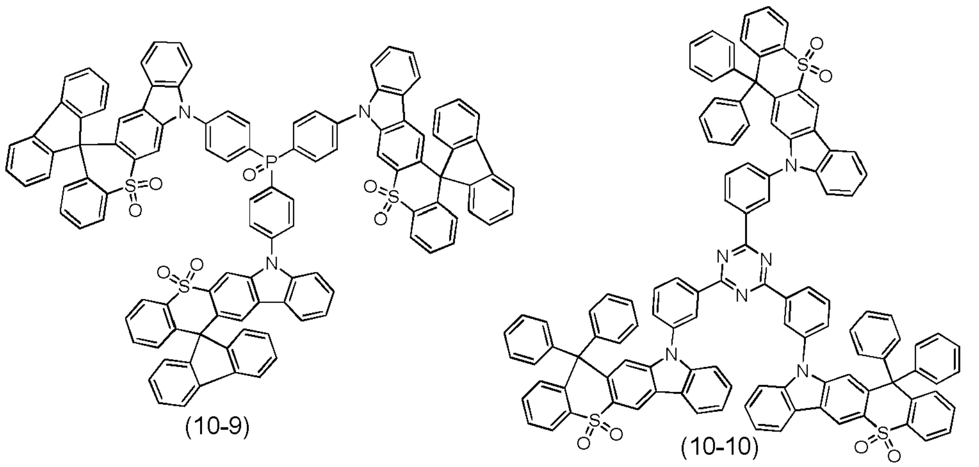

(10-9)

(10-10)

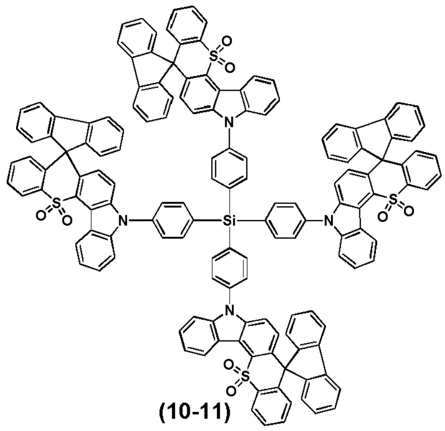

**(10-11)**

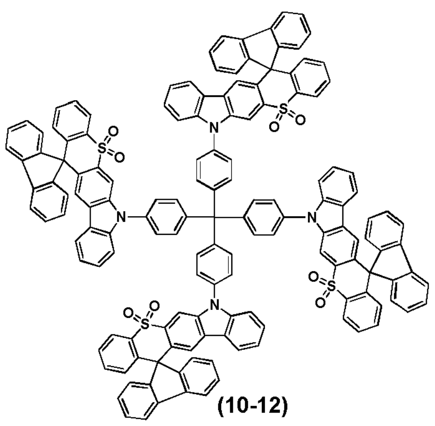

**(10-12)**

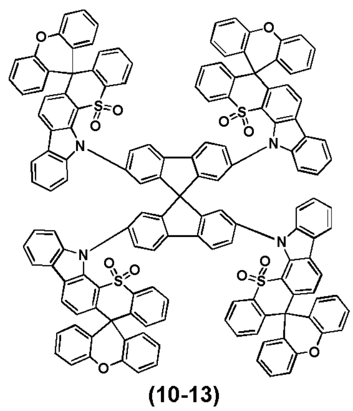

**(10-13)**

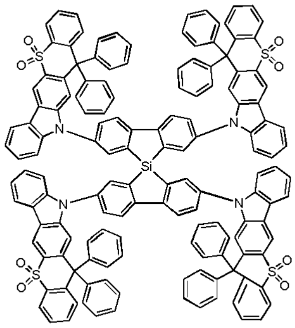

(10-14)

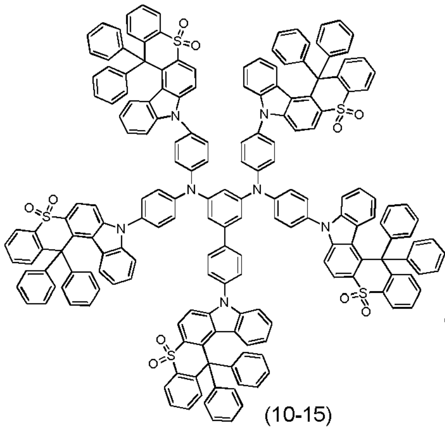

(10-15)

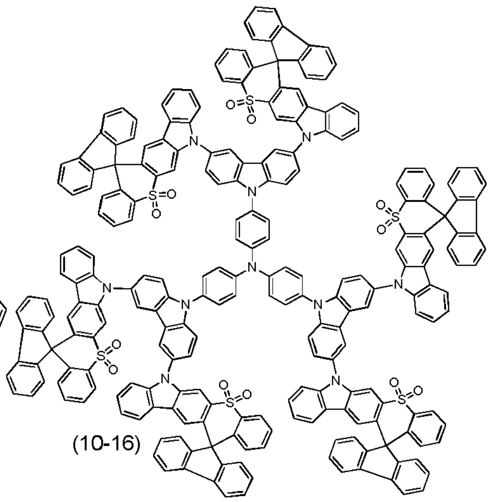

(10-16)

In one embodiment, the above sulfone-containing fused heterocyclic compound is a small molecule material.

The term "small molecule" as defined herein refers to a molecule that is not a polymer, oligomer, dendrimer, or blend. In particular, there are no repeating structures in small molecules. The molecular weight of the small molecule is no greater than 3000 g/mole in one embodiment, no greater than 2000 g/mole in another embodiment, and no greater than 1500 g/mole in a particular embodiment. Polymer includes homopolymer, copolymer, and block copolymer. In addition, in the present disclosure, the polymer also includes dendrimer. The synthesis and application of dendrimers are described in Dendrimers and Dendrons, Wiley-VCH Verlag GmbH & Co. KGaA, 2002, Ed. George R. Newkome, Charles N. Moorefield, Fritz Vogtle.

Conjugated polymer is a polymer whose backbone is primarily consisted of the sp2 hybrid orbital of carbon (C) atom. Some known examples are polyacetylene and poly (phenylene vinylene), on the backbone of which the carbon atom can also be optionally substituted by other non-carbon atoms, and which is still considered to be a conjugated polymer when the sp2 hybridization on the backbone is interrupted by some natural defects. In addition, the conjugated polymer in the present disclosure may also comprise aryl amine, aryl phosphine and other heteroaromatics, organometallic complexes, and the like on the backbone.

Specifically, the present disclosure also relates to a polymer comprising a repeating unit which comprises at least one structural unit shown in general formula (1). In some embodiments, the polymer is a non-conjugated polymer, wherein the structural unit shown in general formula (1) is on the side chain. In another embodiment, the polymer is a conjugated polymer.

The present disclosure further relates to a mixture comprising at least one sulfone-containing fused heterocyclic compound according to the present disclosure, and at least one other organic functional material. Alternatively, the mixture includes at least one polymer of the present disclosure as well as an organic functional material.

The one other organic functional material described herein comprises at least one of a hole (also called electron hole) injection or transport material (HIM/HTM), a hole blocking material (HBM), an electron injection or transport material (EIM/ETM), an electron blocking material (EBM), an organic matrix material (Host), a singlet emitter (fluorescent emitter), a thermally activated delayed fluorescent material (TADF), a triplet emitter (phosphorescent emitter), and a light-emitting organometallic complex. Various organic functional materials are described in detail in, for example, WO2010135519A1, US20090134784A1 and WO 2011110277A1, and the entire contents of these three patent documents are hereby incorporated by reference.

The organic functional materials may be small molecule materials or polymer materials.

In certain embodiments, according to the mixture of the present disclosure, the content of the sulfone-containing fused heterocyclic compound or the polymer in the mixture is 50 wt% to 99.9 wt%, further 60 wt% to 97 wt% in other embodiments, still further 70 wt% to 95 wt% in other embodiments, still further 70 wt% to 90 wt% in other embodiments.

In one embodiment, the mixture according to the present disclosure comprises a compound or a polymer according to the present disclosure and a phosphorescent emitting material.

In another embodiment, the mixture according to the present disclosure comprises a compound or polymer according to the present disclosure and a TADF material.

In another embodiment, the mixture according to the present disclosure comprises a compound or polymer according to the present disclosure, a phosphorescent emitting material and a TADF material.

In another embodiment, the mixture according to the present disclosure comprises a compound or polymer according to the present disclosure, a phosphorescent emitting material and a host material.

In certain embodiments, the mixture according to the present disclosure comprises a compound or polymer

according to the present disclosure and a fluorescent emitting material.

The fluorescent emitting material (singlet emitter), the phosphorescent emitting material (triplet emitter), and the TADF material are described in more detail below (but not limited thereto).

1. Singlet Emitter

The singlet emitter tends to have a longer conjugate π-electron system. To date, there have been many examples, such as, styrylamine and derivatives thereof disclosed in JP2913116B and WO2001021729A1, and indenofluorene and derivatives thereof disclosed in WO2008/006449 and WO2007/140847.

In one embodiment, the singlet emitter can be selected from the group consisting of mono-styrylamine, di-styrylamine,tri-styrylamine, tetra-styrylamine, styrene phosphine, styrene ether, and arylamine.

A mono-styrylamine is a compound comprising an unsubstituted or substituted styryl group and at least one amine, for example an aromatic amine. A di-styrylamine is a compound comprising two unsubstituted or substituted styryl groups and at least one amine, for example an aromatic amine. A tri-styrylamine is a compound comprising three unsubstituted or substituted styryl groups and at least one amine, for example an aromatic amine. A tetra-styrylamine is a compound comprising four unsubstituted or substituted styryl groups and at least one amine, for example an aromatic amine. Particularly, styrene is stilbene, which may be further substituted. The definitions of the corresponding phosphines and ethers are similar to those of amines. An aryl amine or aromatic amine refers to a compound comprising three unsubstituted or optionally substituted aromatic cyclic or heterocyclic systems directly attached to nitrogen. At least one of these aromatic cyclic or heterocyclic systems is selected from fused ring systems in one embodiment and has at least 14 aromatic ring atoms in a particular embodiment. Among the examples are aromatic anthramine, aromatic anthradiamine, aromatic pyrene amines, aromatic pyrene diamines, aromatic chrysene amines and aromatic chrysene diamine. An aromatic anthramine refers to a compound in which a diarylamino group is directly attached to anthracene, at position 9 in a particular embodiment. An aromatic anthradiamine refers to a compound in which two diarylamino groups are directly attached to anthracene, at positions 9, 10 in a particular embodiment. An aromatic pyrene amines, aromatic pyrene diamines, aromatic chrysene amines and aromatic chrysene diamine are similarly defined, wherein the diarylarylamino group is attached to position 1 or 1 and 6 of pyrene in a particular embodiment.

The examples of singlet emitter based on vinylamine and arylamine can be found in the following patent documents: WO2006/000388, WO2006/058737, WO2006/000389, WO2007/065549, WO2007/115610, US7250532 B2, DE 102005058557 A1, CN1583691 A, JP08053397 A, US6251531 B1, US 2006/210830 A, EP1957606 A1 and US2008/0113101 A1, hereby the entire contents of which are incorporated herein by reference.

The examples of singlet emitters based on distyrylbenzene and derivatives thereof can be found in US 5121029.

In some embodiments, the singlet emitter may be selected from the group consisting of: indenofluorene-amine and indenofluorene-diamine such as disclosed in WO 2006/122630, benzoindenofluorene-amine and benzoindenofluorene-diamine such as disclosed in WO 2008/006449, dibenzoindenofluorene-amine and dibenzoindenofluorene-diamine such as disclosed in WO2007/140847.

Other materials useful as singlet emitters include, but not limited to, polycyclic aromatic compounds, especially any one selected from the derivatives of the following compounds: anthracenes such as 9,10-di(2-naphthylanthracene), naphthalene, tetraphenyl, oxyanthene, phenanthrene, perylene (such as 2,5,8,11-tetra-t-butylatedylene), indenoperylene, phenylenes (such as 4,4 '- (bis (9-ethyl-3-carbazovinylene) -1,1'-biphenyl), periflanthene, decacyclene, coronene, fluorene, spirobifluorene, arylpyren (e.g., US20060222886), arylenevinylene (e.g., US5121029, US5130603), cyclopentadiene such as tetraphenylcyclopentadiene, rubrene, coumarine, rhodamine, quinacridone, pyrane such as 4 (dicyanoethylene) -6-(4-dimethylaminostyryl-2-methyl) -4H-pyrane (DCM), thiapyran, bis (azinyl) imine-boron compounds (US 2007/0092753 A1), bis (azinyl) methene compounds, carbostyryl compounds, oxazone, benzoxazole, benzothiazole, benzimidazole, and diketopyrrolopyrrole. Examples of some singlet emitter materials may be found in the following patent documents: US 20070252517 A1, US 4769292, US 6020078, US 2007/0252517 A1, US 2007/0252517 A1, the whole contents of which are incorporated herein by reference.

Examples of suitable singlet emitters are listed below:

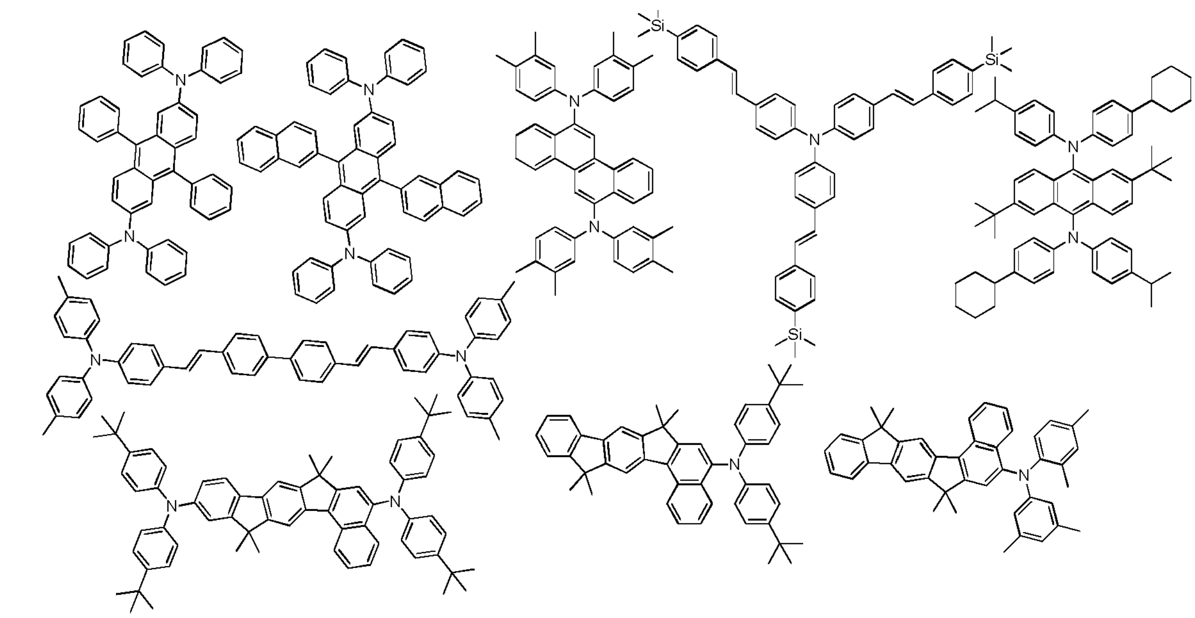

2. Thermally activated delayed fluorescent materials (TADF):

Traditional organic fluorescent materials can only emit light using 25% singlet excitonic luminescenceformed by electrical excitation, and the devices have relatively low internal quantum efficiency (up to 25%). The phosphorescent material enhances the intersystem crossing due to the strong spin-orbit coupling of the heavy atom center, the singlet exciton and the triplet exciton luminescence formed by the electric excitation can be effectively utilized, so that the internal quantum efficiency of the device can reach 100%. However, the phosphor materials are expensive, the material stability is poor, and the device efficiency roll-off is a serious problem, which limit its application in OLED. Thermally-activated delayed fluorescent materials are the third generation of organic light-emitting materials developed after organic fluorescent materials and organic phosphorescent materials. This type of material generally has a small singlet-triplet energy level difference ($\Delta$Est), and triplet excitons can be converted to singlet excitons by intersystem crossing. This can make full use of the singlet excitons and triplet excitons formed under electric excitation. The device can achieve 100% quantum efficiency.

The TADF material needs to have a small singlet-triplet energy level difference, typically $\Delta Est<0.3$ eV in one emdodiment,$\Delta Est<0.2$ eV in another embodiment,$\Delta Est<0.1$ eV in another embodiment, and $\Delta Est<0.05$ eV in a particular embodiment. In one embodiment, TADF has good fluorescence quantum efficiency. Some TADF emitting materials can be found in the following patent documents: CN103483332(A), TW201309696(A), TW201309778(A), TW201343874(A), TW201350558(A), US20120217869(A1), WO2013133359(A1), WO2013154064 ( A1), Adachi, et.al. Adv. Mater., 21, 2009, 4802, Adachi, et.al. Appl. Phys. Lett., 98, 2011, 083302, Adachi, et.al. Appl. Phys. Lett ., 101, 2012, 093306, Adachi, et.al. Chem. Commun., 48, 2012, 11392, Adachi, et.al. Nature Photonics, 6, 2012, 253, Adachi, et.al. Nature, 492, 2012, 234, Adachi, et.al. J. Am. Chem. Soc, 134, 2012, 14706, Adachi, et.al. Angew. Chem. Int. Ed, 51, 2012, 11311, Adachi, et.al. Chem. Commun., 48, 2012, 9580, Adachi, et.al. Chem. Commun., 48, 2013, 10385, Adachi, et.al. Adv. Mater., 25, 2013, 3319, Adachi, et.al . Adv. Mater., 25, 2013, 3707, Adachi, et.al. Chem. Mater., 25, 2013, 3038, Adachi, et.al. Chem. Mater., 25, 2013, 3766, Adachi, et. Al. J. Mater. Chem. C., 1, 2013, 4599, Adachi, et.al. J. Phys. Chem. A., 117, 2013, 5607. The entire contents of the above listed patent or literature documents are hereby incorporated by reference.

Some examples of suitable TADF light-emitting materials are listed below:

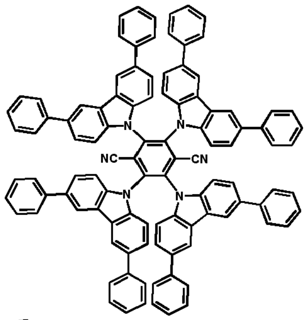
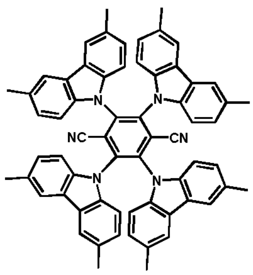
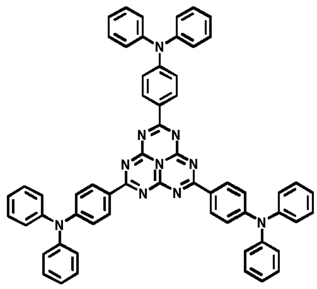
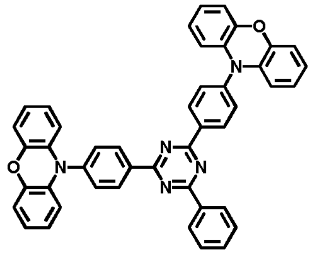
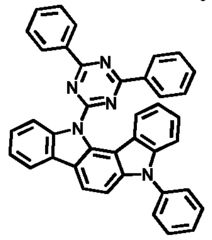
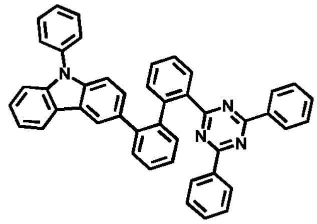
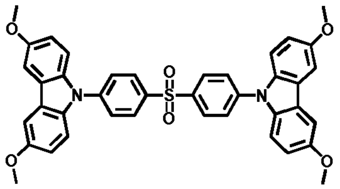
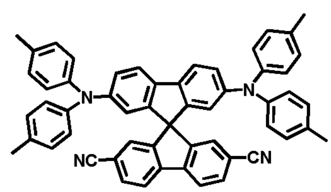
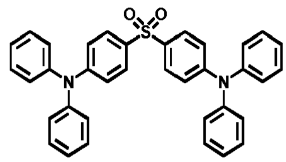
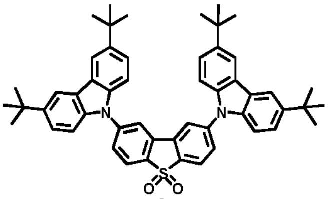
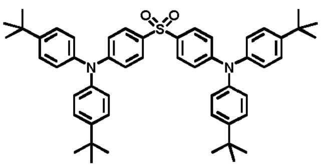
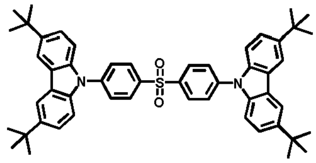
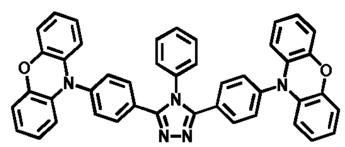
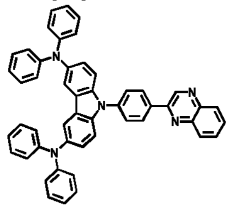
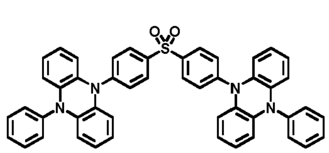
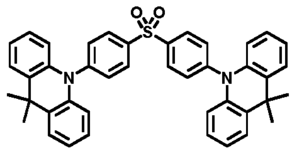
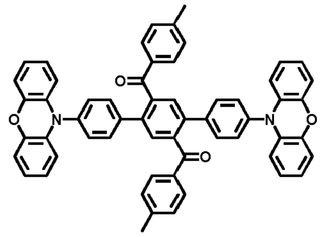
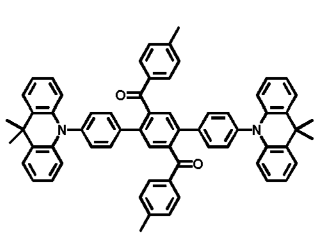
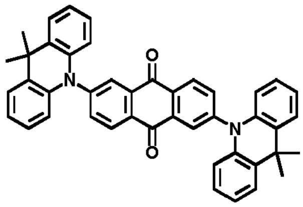
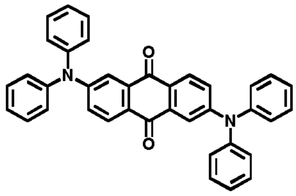

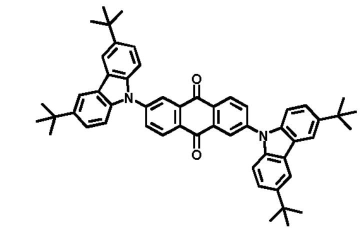
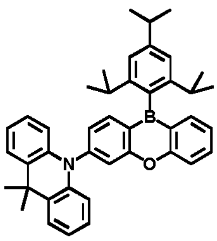
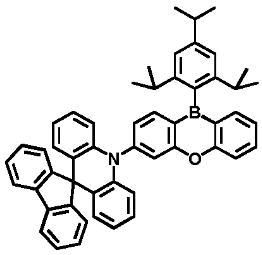
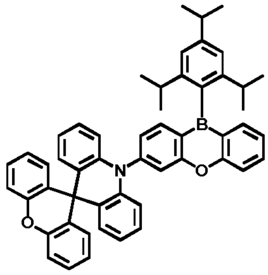
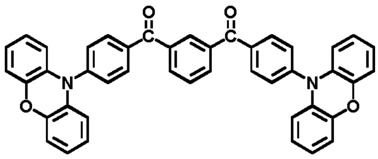
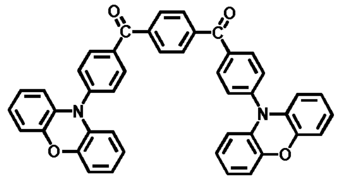
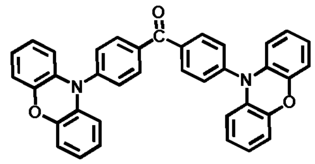
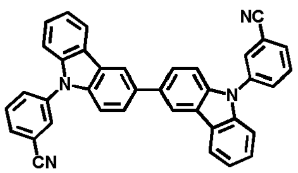
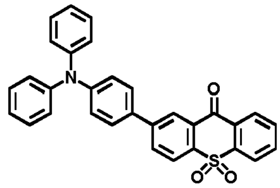
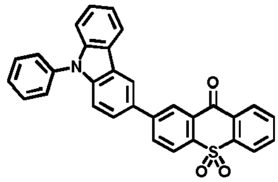
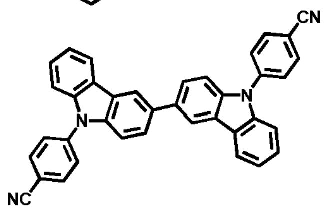
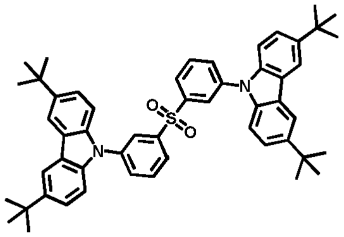
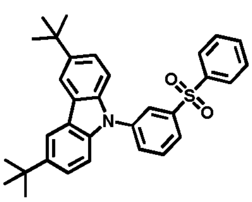

3. Triplet Emitter

Triplet emitters are also called phosphorescent emitters. In one embodiment, the triplet emitter is a metal complex with general formula $M(L)_n$, wherein M is a metal atom, and each occurrence of L may be the same or different and is an organic ligand which is bonded or coordinated to the metal atom M through one or more positions; n is an integer greater than 1, for example 1,2,3,4,5 or 6. Optionally, these metal complexes are attached to a polymer through one or more positions, for example through organic ligands.

In one embodiment, the metal atom M is selected from a transition metal element or a lanthanide element or a lanthanoid element. In one embodiment, the metal atom M is selected from the group consisting of Ir, Pt, Pd, Au, Rh, Ru, Os, Sm, Eu, Gd, Tb, Dy , Re, Cu or Ag. In another embodiment, the metal atom M is selected from the group consisting of Os, Ir, Ru, Rh, Re, Pd or Pt.

In one embodiment, the triplet emitter comprises chelating ligands, i.e. ligands, coordinated with the metal via at least two binding sites. In another embodiment, the triplet emitter comprises two or three identical or different bidentate or multidentate ligands. The chelating ligands are helpful to improve the stability of the metal complexes.

Examples of the organic ligands may be selected from the group consisting of phenylpyridine derivatives, 7,8-benzoquinoline derivatives, 2 (2 -thienyl) pyridine derivatives, 2 (1-naphthyl) pyridine derivatives, and 2 phenylquinoline derivatives. All of these organic ligands may be substituted, for example, substituted by fluoromethyl or trifluoromethyl. Auxiliary ligands may be selected from acetylacetone or picric acid in one embodiment.

In one embodiment, the metal complexes that can be used as triplet emitters have the following form:

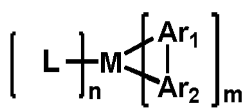

wherein M is a metal and selected from transition metal elements, lanthanoid elements, or lanthanoid elements;

Each occurrence of $Ar_1$ may be the same or different, wherein $Ar_1$ is a cyclic group and comprises at least one donor atom (i.e., an atom having one lone pair of electrons, such as nitrogen or phosphorus) through which the cyclic

group is coordinately coupled with metal; Each occurrence of $Ar_2$ may be the same or different, wherein $Ar_2$ is a cyclic group and comprises at least one carbon atom through which the cyclic group is coupled with metal; $Ar_1$ and $Ar_2$ are covalently bonded together, and each of them may carry one or more substituents, and they may be coupled together by substituents again; Each occurrence of L may be the same or different, wherein L is an auxiliary ligand, and may be a bidentate chelating ligand. In one embodiment, L is a monoanionic bidentate chelating ligand; m is 1, 2 or 3, such as 2 or 3. In one embodiment, m is 3; n is 0, 1 or 2, such as 0 or 1. In one embodiment, n is 0.

Some examples of triplet emitter materials and examples of applications thereof can be found in the following patent documents and references: WO 200070655, WO 200141512, WO 200202714, WO 200215645, EP 1191613, EP 1191612, EP 1191614, WO 2005033244, WO 2005019373, US 2005/0258742, WO 2009146770, WO 2010015307, WO 2010031485, WO 2010054731, WO 2010054728, WO 2010086089, WO 2010099852, WO 2010102709, US 20070087219 A1, US 20090061681 A1, US 20010053462 A1, Baldo, Thompson et al. Nature 403, (2000), 750-753, US 20090061681 A1, US 20090061681 A1, Adachi et al. Appl. Phys. Lett. 78 (2001), 1622-1624, J. Kido et al. Appl. Phys. Lett. 65 (1994), 2124, Kido et al. Chem. Lett. 657, 1990, US 2007/0252517 A1, Johnson et al., JACS 105, 1983, 1795, Wrighton, JACS 96, 1974, 998, Ma et al., Synth. Metals 94, 1998, 245, US 6824895, US 7029766, US 6835469, US 6830828, US 20010053462 A1, WO 2007095118 A1, US 2012004407A1, WO 2012007088A1, WO2012007087A1, WO 2012007086A1, US 2008027220A1, WO 2011157339A1, CN 102282150A, WO 2009118087A1. The entire contents of the above listed patent documents and literatures are hereby incorporated by reference.

Some suitable examples of triplet emitters are listed below:

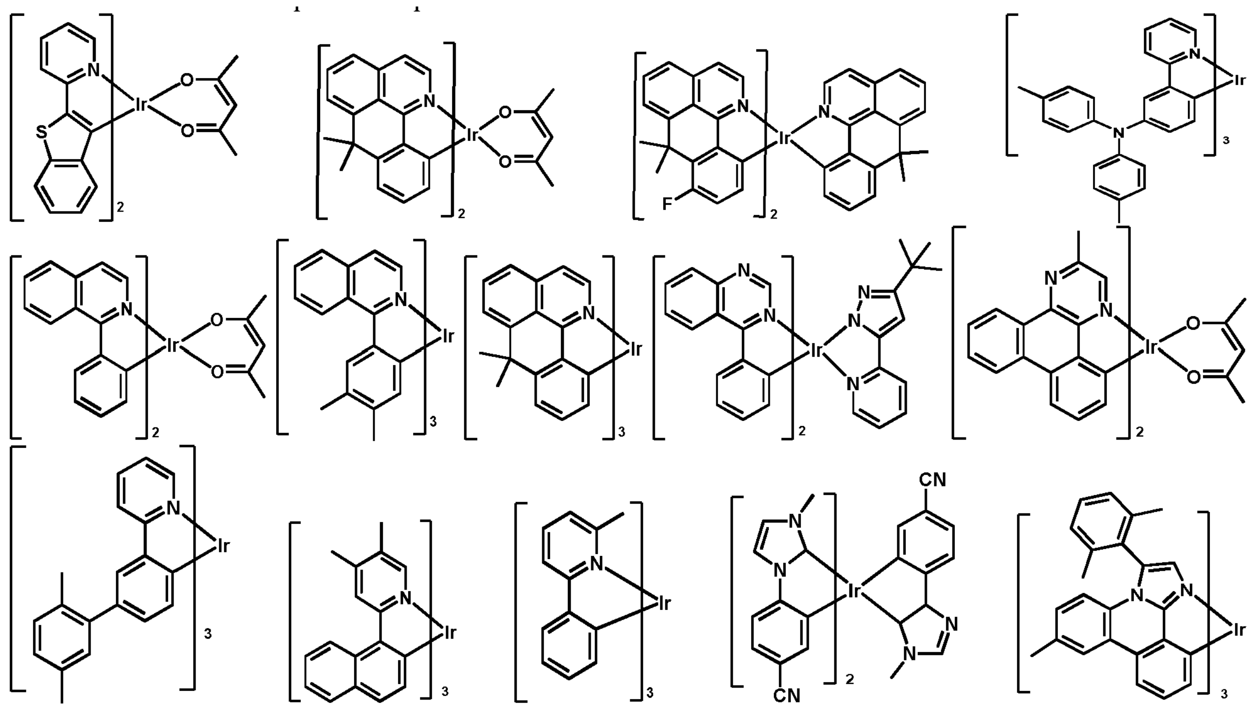

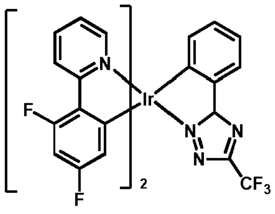 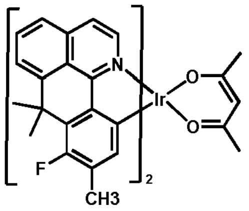 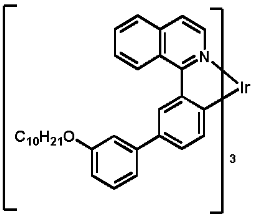 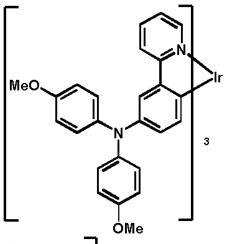 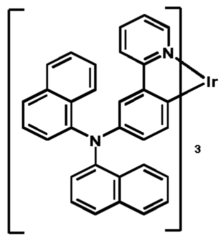

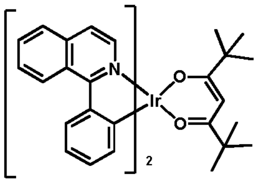 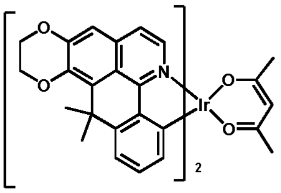 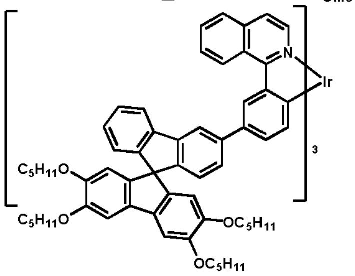 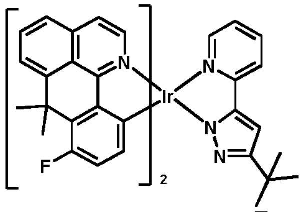

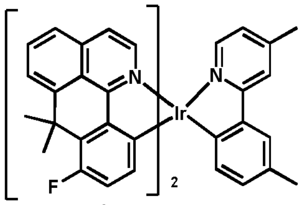 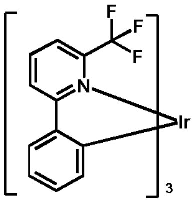 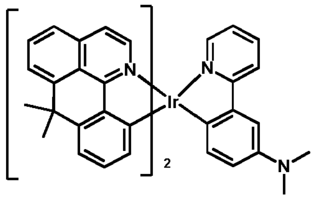 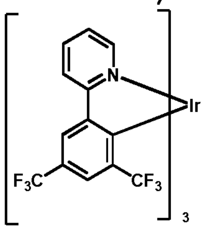

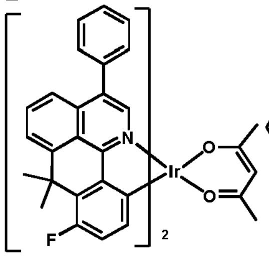 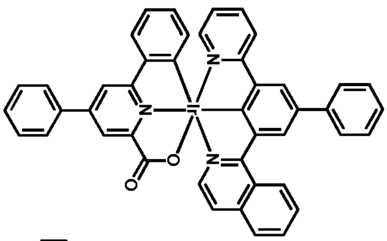 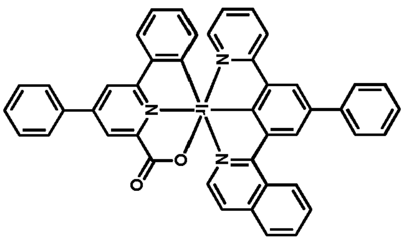 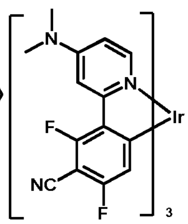

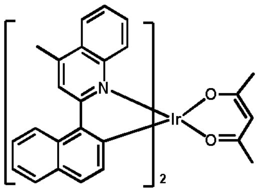 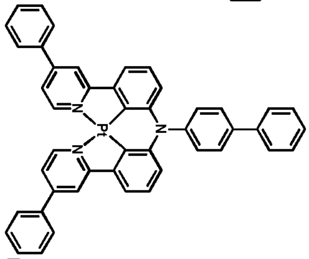 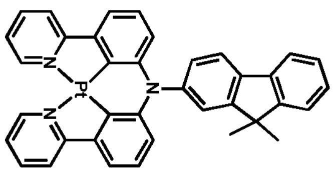 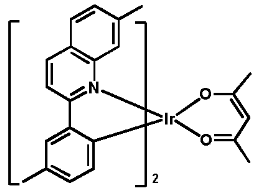

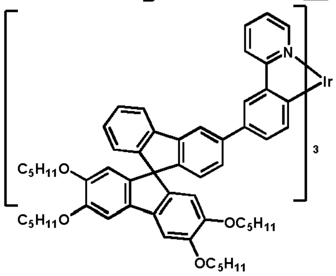 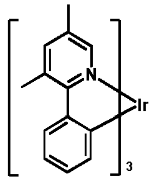 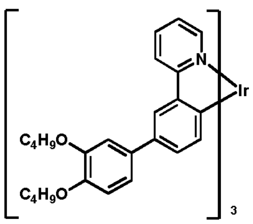 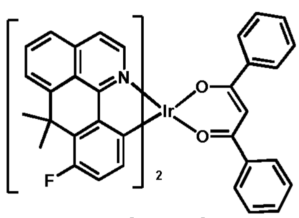

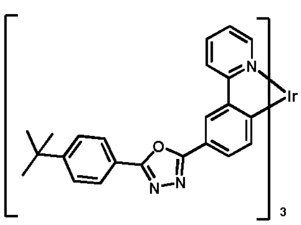 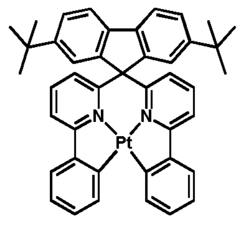 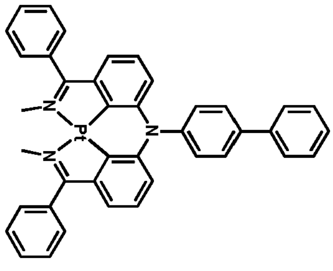 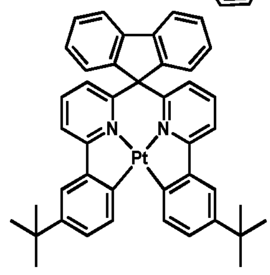

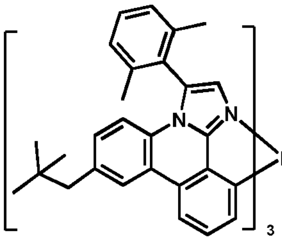 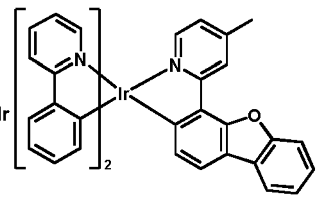 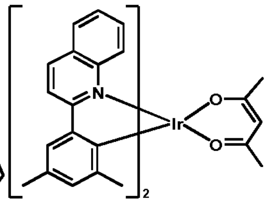

Another purpose of the present disclosure is to provide material solutions for printing OLED.

In certain embodiments, the sulfone-containing fused heterocyclic compound according to the present disclosure has a molecular weight ≥ 700 g/mol, further a molecular weight ≥ 800 g/mol in other embodiments, still further a molecular weight ≥ 900 g/mol in other embodiments, and still further a molecular weight ≥ 1000 g/mol in other embodiments, and still further a molecular weight ≥ 1100 g/mol in other embodiments.

In certain embodiments, the sulfone-containing fused heterocyclic compound according to the present disclosure has a solubility in toluene ≥ 10 mg/ml at 25°C, further a solubility in toluene ≥ 15 mg/ml at 25° C in other embodiments, and still further a solubility in toluene ≥ 20 mg/ml at 25° C in other embodiments.

The present disclosure further relates to a formulation or an ink, and the formulation includes the above sulfone-containing fused heterocyclic compound and at least one organic solvent. Alternatively, the formulation includes the above polymer and at least one organic solvent. Alternatively, the formulation includes the above mixture and at least one organic solvent. The present disclosure further provides a film comprising the compound or polymer according to the present disclosure prepared from a solution.

The viscosity and surface tension of ink are important parameters when the ink is used in the printing process. The suitable surface tension parameters of ink are suitable for a specific substrate and a specific printing method.

In one embodiment, the surface tension of the ink according to the present disclosure at working temperature or at 25°C is in the range of about 19 dyne/cm to 50 dyne/cm. In another embodiment, the surface tension of the ink according to the present disclosure at working temperature or at 25°C is in the range of 22 dyne/cm to 35 dyne/cm. In another embodiment, the surface tension of the ink according to the present disclosure at working temperature or at 25°C is in the range of 25 dyne/cm to 33 dyne/cm.

In another embodiment, the viscosity of the ink according to the present disclosure at the working temperature or at 25° C is in the range of about 1 cps to 100 cps. In another embodiment, the viscosity of the ink according to the present disclosure at the working temperature or at 25° C is in the range of 1 cps to 50 cps. In another embodiment, the viscosity of the ink according to the present disclosure at the working temperature or at 25° C is in the range of 1.5 cps to 20 cps. In another embodiment, the viscosity of the ink according to the present disclosure at the working temperature or at 25° C is in the range of 4.0 cps to 20 cps. The formulation so formulated will be suitable for inkjet printing.

The viscosity can be adjusted by different methods, such as by proper solvent selection and the concentration of functional materials in the ink. The ink according to the present disclosure comprising the compound or polymer can facilitate the adjustment of the printing ink in an appropriate range according to the printing method used. In general, the weight ratio of the functional material contained in the formulation according to the disclosure is in the range of 0.3 wt% to 30 wt%. In one embodiment, the weight ratio of the functional material contained in the formulation according to the disclosure is in the range of 0.5 wt% to 20 wt%. In another embodiment, the weight ratio of the functional material contained in the formulation according to the disclosure is 0.5 wt% to 15 wt%. In another embodiment, the weight ratio of the functional material contained in the formulation according to the disclosure is in the range of 0.5 wt% to 10 wt%. In another embodiment, the weight ratio of the functional material contained in the formulation according to the disclosure is in the range of 1 wt% to 5 wt%.

In some embodiments, according to the ink of the present disclosure, the at least one organic solvent is selected from solvents based on aromatics or heteroaromatics, especially aliphatic chain/ring substituted aromatic solvents, or aromatic ketone solvents, or aromatic ether solvents.

Examples suitable for solvents of the present disclosure include, but not limited to, the solvents based on aromatics or heteroaromatics: p-diisopropylbenzene, pentylbenzene, tetrahydronaphthalene, cyclohexyl benzene, chloronaphthalene, 1,4-dimethylnaphthalene, 3-isopropylbiphenyl, p-cymene, dipentylbenzene, tripentylbenzene, pentyltoluene, o-xylene, m-xylene, p-xylene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, p-diisopropylbenzene, 1-methoxynaphthalene, cyclohexylbenzene, dimethylnaphthalene, 3-isopropylbiphenyl, p-cymene, 1-methylnaphthalene, 1,2,4-trichlorobenzene, 1,3 -dipropoxybenzene, 4,4-difluorodiphenylmethane, 1,2-dimethoxy-4-(1-propenyl)benzene, diphenylmethane, 2-phenylpyridine, 3-phenylpyridine, N-methyldiphenylamine 4-isopropylbiphenyl, α,α--dichlorodiphenylmethane, 4-(3-phenylpropyl)pyridine, benzylbenzoate, 1,1-di(3,4-dimethylphenyl)ethane, 2-isopropylnaphthalene, dibenzylether, and the like.; solvents based on ketones: 1-tetralone, 2-tetralone,

2-(phenylepoxy)tetralone, 6-(methoxyl)tetralone, acetophenone, phenylacetone, benzophenone , and derivatives thereof, such as 4-methylacetophenone, 3-methylacetophenone, 2-methylacetophenone, 4-methylphenylacetone, 3-methylphenylacetone, 2-methylphenylacetone, isophorone, 2,6,8-trimethyl-4-nonanone, fenchone, 2-nonanone, 3-nonanone, 5-nonanone, 2-demayone, 2,5-hexanedione, phorone, di-n-amyl ketone; aromatic ether solvents: 3-phenoxytoluene, butoxybenzene, benzylbutylbenzene, p-anisaldehyde dimethyl acetal, tetrahydro-2-phenoxy-2H-pyran, 1,2-dimethoxy 4-(1-propenyl)benzene, 1,4-benzodioxane, 1,3-dipropylbenzene, 2,5-dimethoxytoluene, 4-ethylphenetole, 1,2,4-trimethoxybenzene, 4-(1-propenyl)-1,2-dimethoxybenzene, 1,3-dimethoxybenzene, glycidyl phenyl ether, dibenzyl ether, 4-tert-butylanisole, trans-p-propenylanisole, 1,2-dimethoxybenzene, 1-methoxynaphthalene, diphenyl ether, 2-phenoxymethyl ether, 2-phenoxytetrahydrofuran, ethyl-2-naphthyl ether, pentyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethylene glycol ethyl methyl ether, triethylene glycol butyl methyl ether, tripropylene glycol dimethyl ether, tetraethylene glycol dimethyl ether; and ester solvents: alkyl octoate, alkyl sebacate, alkyl stearate, alkyl benzoate, alkyl phenylacetate, alkyl cinnamate, alkyl oxalate, alkyl maleate, alkyl lactone, alkyl oleate, and the like.

Furthermore, according to the ink of the present disclosure, the at least one solvent can be selected from the group consisting of aliphatic ketones, such as 2-nonanone, 3-nonanone, 5-nonanone, 2-demayone, 2,5-hexanedione, 2,6,8-trimethyl-4-demayone, phorone, di-n-pentyl ketone, and the like; or aliphatic ethers, such as amyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethyl ether alcohol ethyl methyl ether, triethylene glycol butyl methyl ether, tripropylene glycol dimethyl ether, tetraethylene glycol dimethyl ether, and the like.

In other embodiments, the printing ink further comprises another organic solvent. Examples of another organic solvent comprise, but not limited to, methanol, ethanol, 2-methoxyethanol, dichloromethane, trichloromethane, chlorobenzene, o-dichlorobenzene, tetrahydrofuran, anisole, morpholine, toluene, o-xylene, m-xylene, p-xylene, 1,4-dioxane, acetone, methyl ethyl ketone, 1,2-dichloroethane, 3-phenoxy toluene, 1,1,1-trichloroethane, 1,1,2,2-tetrachloroethane, ethyl acetate, butyl acetate, dimethylformamide, dimethylacetamide, dimethyl sulfoxide, tetrahydronaphthalene, decalin, indene, and/or mixtures thereof.In one embodiment, the formulation according to the present disclosure is a solution.

In another embodiment, the formulation according to the present disclosure is a suspension.

The present disclosure further relates to the application of the formulation as the printing ink to make an organic electron device, especially by a printing method or a coating method.

The appropriate printing technology or coating technology includes, but is not limited to inkjet printing, nozzle printing, typography, screen printing, dip coating, spin coating, blade coating, roller printing, twist roller printing, lithography, flexography, rotary printing, spray coating, brush coating or transfer printing, nozzle printing, slot die coating, and the like. The first preference is inkjet printing, slot die coating, nozzle printing, and typography. The solution or the suspension liquid may further includes one or more components, such as a surfactant compound, a lubricant, a wetting agent, a dispersant, a hydrophobic agent, a binder, to adjust the viscosity and the film forming property and to improve the adhesion property. The detailed information relevant to the printing technology and requirements of the printing technology to the solution, such as solvent, concentration, and viscosity, may be referred to Handbook of Print Media: Technologies and Production Methods, Helmut Kipphan, ISBN 3-540-67326-1.

Based on the above sulfone-containing fused heterocyclic compound, the present disclosure also provides an application of the above sulfone-containing fused heterocyclic compound or polymer in organic electronic devices. The organic electronic devices may be selected from, but not limited to, an organic light-emitting diode (OLED), an organic photovoltaic cell (OPV), an organic light-emitting electrochemical cell (OLEEC), an organic field effect transistor (OFET), an organic light-emitting field effect transistor, an organic laser, an organic spintronic device, organic sensor, and an organic plasmon emitting diode, and the like, specially OLED. In an embodiment of the present disclosure, the organic compound is used in the light-emitting layer of the OLED device.

The present disclosure further relates to an organic electronic device comprising at least one sulfone-containing fused heterocyclic compound, polymer or mixture as described above. Generally, such organic electronic device comprises at least one cathode, one anode, and at least one functional layer located between the cathode and the anode, wherein the functional layer comprises at least one compound or polymer as described above. The organic electronic devices may be selected from, but not limited to, an organic light-emitting diode (OLED), an organic photovoltaic cell (OPV), an organic light-emitting electrochemical cell (OLEEC), an organic field effect transistor (OFET), an organic light-emitting field effect transistor, an organic laser, an organic spintronic device, an organic sensor, and an organic plasmon emitting diode.

In one embodiment, the organic electronic device is an electroluminescent device, in particular an OLED, comprising a substrate, an anode, at least one light-emitting layer, a cathode, optionally comprising a hole transport layer. In some embodiments, the hole transport layer comprises the sulfone-containing fused heterocyclic compound or the polymer according to the present disclosure. In one embodiment, the light-emitting layer comprises the sulfone-containing fused heterocyclic compound or the polymer according to the present disclosure, further, the light-emitting layer comprises the sulfone-containing fused heterocyclic compound or the polymer according to the present disclosure

and at least one light-emitting material which may be selected from a fluorescent emitter, a phosphorescent emitter, a TADF material or a light-emitting quantum dot.

The structure of the electroluminescent device is described below, but it is not limited.

The substrate may be opaque or transparent. The transparent substrate may be used to make the transparent luminescent device, which may be referred to, for example, Bulovic et al., Nature, 1996, 380, page 29 and Gu et al., Appl. Phys. Lett., 1996, 68, page2606. The substrate may be rigid or elastic. The substrate may be plastic, metal, a semiconductor wafer, or glass. In one embodiment, the substrate has a smooth surface. The substrate without any surface defects is the particular ideal selection. In one embodiment, the substrate is flexible and may be selected from a polymer thin film or a plastic which have the glass transition temperature Tg larger than 150°C, larger than 200°C in one embodiment, larger than 250°C in another embodiment, larger than 300°C in a particular embodiment. Suitable examples of the flexible substrate are polyethylene terephthalate (PET) and polyethylene 2,6-naphthalate (PEN).

The anode may include a conductive metal, metallic oxide, or a conductive polymer. The anode can inject holes easily into the hole injection layer (HIL), the hole transport layer (HTL), or the light-emitting layer. In one embodiment, the absolute value of the difference between the work function of the anode and the HOMO energy level or the valence band energy level of the emitter in the light-emitting layer or of the p-type semiconductor material of the HIL or HTL or the electron blocking layer (EBL) is smaller than 0.5 eV in one embodiment, smaller than 0.3 eV in another embodiment, smaller than 0.2 eV in a particular embodiment. Examples of the anode material include, but are not limited to Al, Cu, Au, Ag, Mg, Fe, Co, Ni, Mn, Pd, Pt, ITO, aluminum-doped zinc oxide (AZO), and the like. Other suitable anode materials are known and may be easily selected by one of ordinary skilled in the art. The anode material may be deposited by any suitable technologies, such as the suitable physical vapor deposition method which includes a radio frequency magnetron sputtering, a vacuum thermal evaporation, an electron beam, and the like. In some embodiments, the anode is patterned and structured. A patterned ITO conductive substrate may be purchased from market to prepare the device according to the present disclosure.

The cathode may include a conductive metal or metal oxide. The cathode can inject electrons easily into the electron injection layer (EIL) or the electron transport layer (ETL), or directly injected into the light-emitting layer. In one embodiment, the absolute value of the difference between the work function of the cathode and the LUMO energy level or the valence band energy level of the emitter in the light-emitting layer or of the n type semiconductor material as the electron injection layer (EIL) or the electron transport layer (ETL) or the hole blocking layer (HBL) is smaller than 0.5 eV in one embodiment, smaller than 0.3 eV in another embodiment, smaller than 0.2 eV in another embodiment. In principle, all materials capable of using as the cathode of the OLED may be used as the cathode material of the device of the present disclosure. Examples of the cathode material include, but are not limited to, Al, Au, Ag, Ca, Ba, Mg, LiF/Al, MgAg alloy, $BaF_2$/Al, Cu, Fe, Co, Ni, Mn, Pd, Pt, ITO, and the like. The cathode material may be deposited by any suitable technologies, such as the suitable physical vapor deposition method which includes a radio frequency magnetron sputtering, a vacuum thermal evaporation, an electron beam, and the like.

OLED can also comprise other functional layers such as a hole injection layer (HIL), a hole transport layer (HTL), an electron blocking layer (EBL), an electron injection layer (EIL), an electron transport layer (ETL), and a hole blocking layer (HBL). Materials suitable for use in these functional layers are readily available in related patents or literature.

In one embodiment, in the organic light-emitting device according to the present disclosure, the light-emitting layer comprises the sulfone-containing fused heterocyclic compound or the polymer of the present disclosure and is prepared by vacuum evaporation or solution processing method, such as by a solution processing method.

The light-emitting wavelength of the light-emitting device according to the present disclosure is between 300 and 1000 nm. In one embodiment, the light-emitting wavelength of the light-emitting device according to the present disclosure is between 350 and 900 nm. In another embodiment, the light-emitting wavelength of the light-emitting device according to the present disclosure is between 400 and 800 nm.

The present disclosure also relates to the application of the organic electronic device according to the present disclosure in various electronic equipment, including, but not limited to display equipments, lighting equipments, light sources, and sensors, and the like.

The present disclosure will be described below with reference to the preferred embodiments, but the present disclosure is not limited to the following embodiments. It should be understood that the appended claims summarized the scope of the present disclosure. Those skilled in the art should realize that certain changes to the embodiments of the present disclosure that are made under the guidance of the concept of the present disclosure will be covered by the spirit and scope of the claims of the present disclosure.

## DETAILED EXAMPLES

1. Preparation of sulfone-containing fused heterocyclic compounds

EXAMPLE 1 (Reference Example)

Synthesis of compound (2-6) with structural formula as follows:

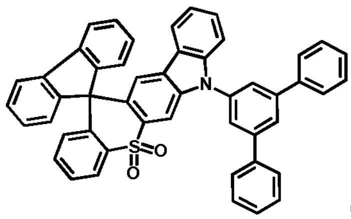

(2-6).

1) Compound (2-6-1) (21.2 g, 100 mmol), ferric chloride (0.64 g, 4 mmol) and chloroform (400 mL) were added to a 1000 mL three-necked flask, stirred, and chloroform solution of liquid bromine (16 g, 100 mmol) was slowly added dropwise at room temperature, after the addition, the mixture was heated to 60°C and reacted under stirring for 12 hours, then the reaction was ended. The reaction was quenched with aqueous solution of sodium bisulfite, then the reaction solution was filtered with suction and the filter residue was recrystallized with 1,4-dioxane, with a yield of 50%. Reaction formula is as follows:

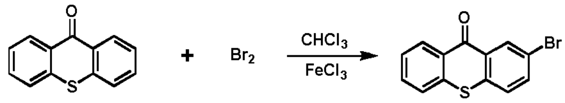


2-6-1 2-6-2

2) Under nitrogen atmosphere, compound (2-6-2) (11.6 g, 40 mmol) and 40 mL of anhydrous THF were added to a 1000 mL three-necked flask, stirred, and THF solution of previously prepared Grignard reagent (2-6-3)(60 mmol) was slowly added, after the addition, the mixture was heated to 60°C and reacted under stirring for 12 hours, 100 mL of deionized water was slowly added dropwise, and the reaction was continued for 1 hour, then the reaction was ended. The reaction solution was rotary evaporated to remove most of the solvent, and then dissolved with dichloromethane and washed with water for 3 times. The organic solution was collected and then mixed with silica gel and purified by column chromatography, with a yield of 60%. Reaction formula is as follows:

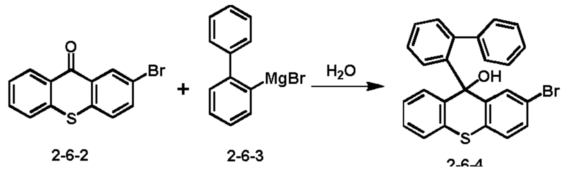


2-6-2 2-6-3 2-6-4

3) Compound (2-6-4) (8.9 g, 20 mmol), 40 mL of acetic acid and 20 mL of hydrobromic acid were added to a 100 mL two-necked flask, and the mixture was heated to 110° C and reacted under stirring for 12 hours, then the reaction was ended. The reaction solution was poured into 300 mL of deionized water and filtered with suction. The filter residue was collected and then recrystallized with ethanol, with a yield of 85%. Reaction formula is as follows:

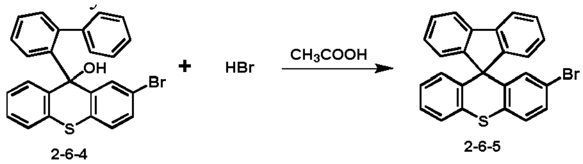


2-6-4 2-6-5

4) Compound (2-6-5) (4.26 g, 10 mmol), bis(pinacolate)diboron (3.05 g, 12 mmol), Pd(dppf)$Cl_2$ (0.05 mmol), potas-

sium acetate (20 mmol) and 60 mL of 1,4-dioxane were added into a 100 mL two-necked flask, and the mixture was heated to 100° C and reacted under stirring for 12 hours, and then the reaction was ended. The reaction solution was added to 400 mL of water and filtered with suction. The filter residue was collected and then mixed with silica gel and purified by column chromatography, with a yield of 85%. Reaction formula is as follows:

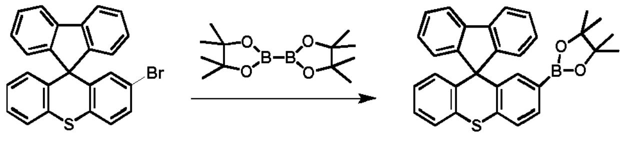

2-6-5 2-6-6

5) Under nitrogen atmosphere, compound (2-6-6) (2.84 g, 6 mmol) and compound (2-6-7) (1.21 g, 6 mmol) , sodium carbonate (1.6 g, 15 mmol), tetrabutylammonium bromide ( 0.48 g, 1.5 mmol) and tetrakis(triphenylphosphine)palladium (0.52 g, 0.45 mmol), 60 mL of 1,4-dioxane and 10 mL of water were added to a 150 mL two-necked flask, and the mixture was heated to 90° C and reacted under stirring for 12 hours, and then the reaction was ended. The reaction solution was added into 300 mL of water, and then extracted and dissolved with dichloromethane and washed with water for 3 times. The organic solution was collected and then mixed with silica gel and purified by column chromatography, with a yield of 85%. Reaction formula is as follows:

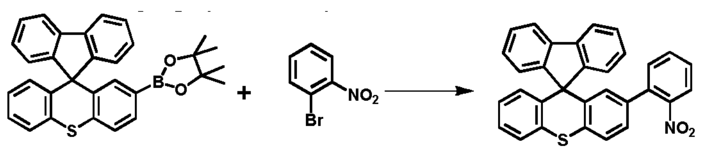

2-6-6 2-6-7 2-6-8

6) Under nitrogen atmosphere, compound (2-6-8) (1.9 g, 4 mmol) and triethylphosphine (2.0 g, 20 mmol) were added to a 100 mL two-necked flask, and the mixture was heated to 190° C and reacted under stirring for 12 hours, and then the reaction was ended. The reaction solution was distilled under reduced pressure to remove most of the solvent, and then dissolved with dichloromethane and washed with water for 3 times. The organic solution was collected and then mixed with silica gel and purified by column chromatography, with a yield of 60%. Reaction formula is as follows:

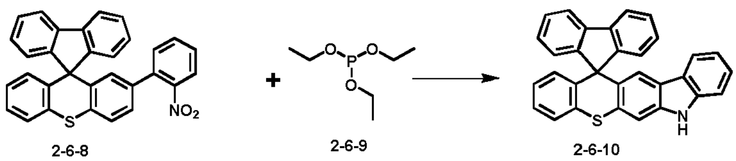

2-6-8 2-6-9 2-6-10

7) Under nitrogen atmosphere, compound (2-6-10) (0.87 g, 2 mmol) and compound (2-6-11) (0.93 g, 3 mmol), copper powder (0.013 g, 0.2 mmol), potassium carbonate ( 0.55 g, 4 mmol) and 18-crown-6 (0.053 g, 0.1 mmol) and o-dichlorobenzene (20 mL) were added to a 100 mL two-necked flask, and the mixture was heated to 150° C and reacted under stirring for 24 hours, and then the reaction was ended. The reaction solution was distilled under reduced pressure to remove most of the solvent, and then dissolved with dichloromethane and washed with water for 3 times. The organic solution was collected and then mixed with silica gel and purified by column chromatography, with a yield of 90%. Reaction formula is as follows:

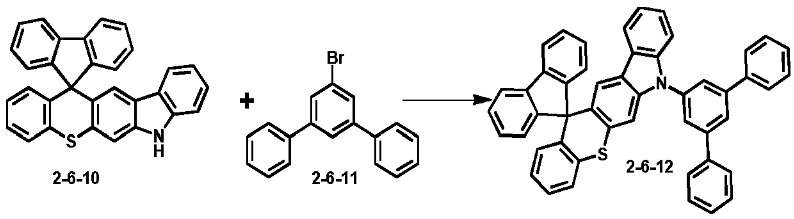

2-6-10 2-6-11 2-6-12

8) Compound (2-6-12) (0.33 g, 0.5 mmol), 15 mL of acetic acid and 2 mL of hydrogen peroxide were added to a

100 mL two-necked flask, and the mixture was heated to 110° C and reacted under stirring for 6 hours, then the reaction was ended. The reaction solution was poured into 100 mL of deionized water and filtered with suction. The filter residue was recrystallized with THF/ethanol solution, with a yield of 90%. Reaction formula is as follows:

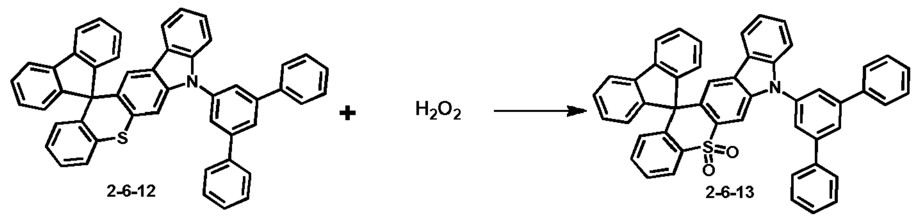

2-6-12
2-6-13

EXAMPLE 2

Synthesis of compound (9-7) with structural formula as follows:

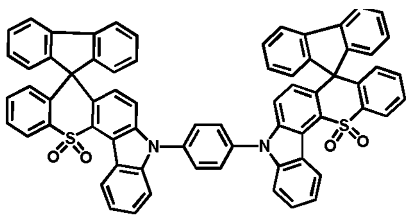
(9-7).

1) Under nitrogen atmosphere, compound (9-7-1) (11.05g, 50 mmol) and 200 mL of anhydrous THF were added to a 500 mL three-necked flask, stirred, and THF solution of previously prepared Grignard reagent (9-7-2)(75 mmol) was slowly added, after the addition, the mixture was heated to 60°C and reacted under stirring for 12 hours, 100 mL of deionized water was slowly added dropwise, and the reaction was continued for 1 hour, then the reaction was ended. The reaction solution was rotary evaporated to remove most of the solvent, and then dissolved with dichloromethane and washed with water for 3 times. The organic solution was collected and then mixed with silica gel and purified by column chromatography, with a yield of 60%. Reaction formula is as follows:

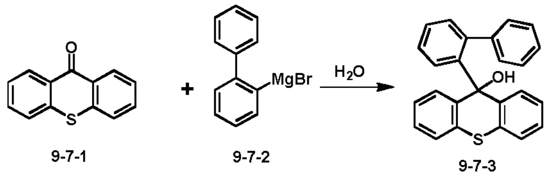

9-7-1
9-7-2
9-7-3

2) Compound (9-7-3) (7.3 g, 20 mmol), 30 mL of acetic acid and 15 mL of hydrobromic acid were added to a 100 mL two-necked flask, and the mixture was heated to 110° C and reacted under stirring for 12 hours, then the reaction was ended. The reaction solution was poured into 300 mL of deionized water and filtered with suction. The filter residue was collected and then recrystallized with ethanol, with a yield of 85%. Reaction formula is as follows:

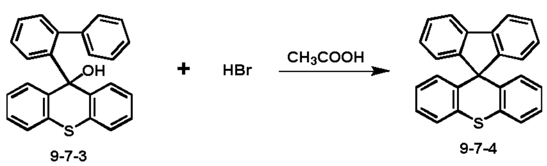

9-7-3
9-7-4

3) Under nitrogen atmosphere, compound (9-7-4) (5.2 g, 15 mmol) and 150 mL of anhydrous THF were added to a 300 mL three-necked flask, stirred, cooled to -78°C, and n-butyllithium (15 mmol) was slowly added dropwise, the mixture was reacted under stirring for 1.5 hours, then THF solution of NBS (2.67 g, 15 mmol) was slowly added dropwise, and the reaction was allowed to be warmed up naturally. The reaction was ended after 12 hours of reaction. Water was added to the reaction solution to quench the reaction. The reaction solution was distilled under reduced pressure to remove most of the solvent and then dissolved with dichloromethane and washed with water for 3 times. The organic solution was collected and spin dried and then recrystallized with a mixed solution of dichloromethane/ethanol, with a yield of 80%. Reaction formula is as follows:

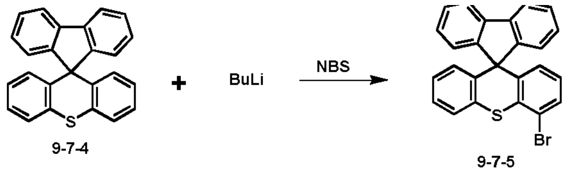


9-7-4 9-7-5

4) Compound (9-7-5) (4.26 g, 10 mmol), bis(pinacolate)diboron (3.05 g, 12 mmol), Pd(dppf)$C_{12}$ (0.05 mmol), potassium acetate (20 mmol) and 60 mL of 1,4-dioxane were added into a 100 mL two-necked flask, and the mixture was heated to 100° C and reacted under stirring for 12 hours, and then the reaction was ended. The reaction solution was added to 400 mL of water and filtered with suction. The filter residue was collected and then mixed with silica gel and purified by column chromatography, with a yield of 80%. Reaction formula is as follows:

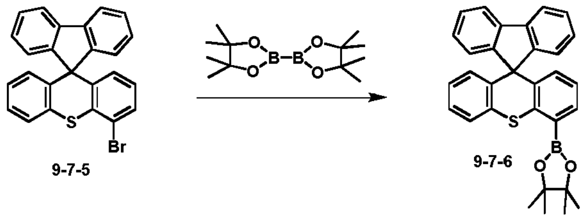

9-7-5 9-7-6

5) Under nitrogen atmosphere, compound (9-7-6) (2.84 g, 6 mmol) and compound (9-7-7) (1.21 g, 6 mmol) , sodium carbonate (1.6 g, 15 mmol), tetrabutylammonium bromide ( 0.48 g, 1.5 mmol) and tetrakis(triphenylphosphine)palladium (0.52 g, 0.45 mmol), 60 mL of 1,4-dioxane and 10 mL of water were added to a 150 mL two-necked flask, and the mixture was heated to 90° C and reacted under stirring for 12 hours, and then the reaction was ended. The reaction solution was added into 300 mL of water, and then extracted and dissolved with dichloromethane and washed with water for 3 times. The organic solution was collected and then mixed with silica gel and purified by column chromatography, with a yield of 90%. Reaction formula is as follows:

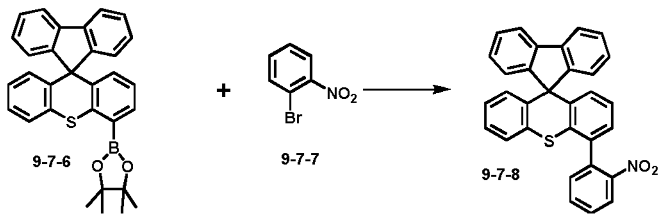

9-7-6 9-7-7 9-7-8

6) Under nitrogen atmosphere, compound (9-7-8) (1.9 g, 4 mmol) and triethylphosphine (2.0 g, 20 mmol) were added to a 100 mL two-necked flask, and the mixture was heated to 190° C and reacted under stirring for 12 hours, and then the reaction was ended. The reaction solution was distilled under reduced pressure to remove most of the solvent, and then dissolved with dichloromethane and washed with water for 3 times. The organic solution was collected and then mixed with silica gel and purified by column chromatography, with a yield of 85%. Reaction formula is as follows:

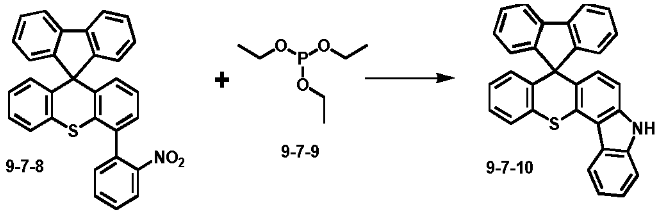

9-7-8 9-7-9 9-7-10

7) Under nitrogen atmosphere, compound (9-7-10) (0.87 g, 2 mmol) and compound (9-7-11) (0.66 g, 2 mmol) , copper powder (0.013 g, 0.2 mmol), potassium carbonate ( 0.55 g, 4 mmol) and 18-crown-6 (0.053 g, 0.1 mmol) and o-dichlorobenzene (20 mL) were added to a 100 mL two-necked flask, and the mixture was heated to 150° C and reacted under stirring for 24 hours, and then the reaction was ended. The reaction solution was distilled under reduced pressure to remove most of the solvent, and then dissolved with dichloromethane and washed with water for 3 times. The organic solution was collected and then mixed with silica gel and purified by column chromatography,

with a yield of 60%.

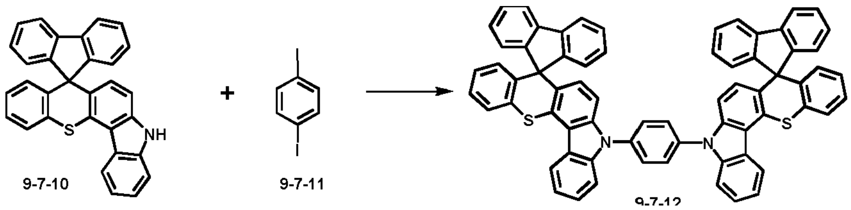

9-7-10 9-7-11 9-7-12

8) Compound (9-7-12) (0.47 g, 0.5 mmol), 15 mL of acetic acid and 5 mL of hydrogen peroxide were added to a 50 mL two-necked flask, and the mixture was heated to 110° C and reacted under stirring for 6 hours, then the reaction was ended. The reaction solution was poured into 100 mL of deionized water and filtered with suction. The filter residue was recrystallized with THF/ethanol solution, with a yield of 90%.

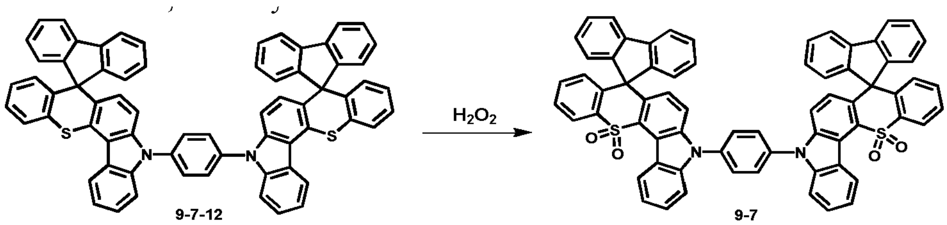


9-7-12 9-7

2. Energy Structure of Organic Compounds

The energy levels of organic materials can be obtained by quantum calculations, such as using TD-DFT (Time Dependent-Density Functional Theory) by Gaussian03W (Gaussian Inc.), and the specific simulation methods can be found in WO2011141110. Firstly, the molecular geometry is optimized by semi-empirical method "Ground State/Semi-empirical/Default Spin/AMI" (Charge 0/Spin Singlet), and then the energy structure of organic molecules is calculated by TD-DFT (time-density functional theory) "TD-SCF/DFT/Default Spin/B3PW91" and the basis set "6-31G (d)" (Charge 0/Spin Singlet). The HOMO and LUMO levels are calculated according to the following calibration formulas, S1 and T1 are used directly.

$$\mathrm{HOMO(eV)} = ((\mathrm{HOMO(G)}\times 27.212)-0.9899)/1.1206$$

$$\mathrm{LUMO(eV)} = ((\mathrm{LUMO(G)}\times 27.212)-2.0041)/1.385$$

wherein HOMO(G) and LUMO(G) in the unit of Hartree are the direct calculation results of Gaussian 03W. The results were shown in Table 1.

Table 1: Energy Structure of Organic Compounds

| Materials | HOMO [eV] | LUMO [eV] | $T_1$ [eV] | $S_1$ [eV] |
|---|---|---|---|---|
| HATCN | -9.04 | -5.08 | 2.32 | 3.17 |
| NPB | -6.72 | -2.85 | 2.97 | 3.46 |
| TCTA | -5.34 | -2.20 | 2.73 | 3.42 |
| 2-6* | -5.90 | -2.58 | 2.93 | 3.05 |
| 9-7 | -5.82 | -2.61 | 2.93 | 2.95 |
| $Ir(ppy)_3$ | -5.30 | -2.35 | 2.70 | 2.93 |
| B3PYMPM | -5.33 | -2.20 | 2.72 | 3.28 |
| *Reference Example | | | | |

$\Delta E(S_1-T_1)$ of compound 2-6 = 0.12eV, $\Delta E(S_1-T_1)$ of compound 9-7 = 0.02eV. The HOMO electron cloud distribution diagram and the LUMO electron cloud distribution diagram of the compound 2-6 prepared in Example 1 are shown in FIG. 1 and FIG. 2, respectively. The HOMO electron cloud distribution diagram and the LUMO electron cloud distribution diagram of the compound 9-7 prepared in Example 2 are shown in FIG. 3 and FIG. 4, respectively. It can be seen from the figures that the HOMO and LUMO of the two compounds have sufficient overlap.

3. Preparation and Characterization of OLED Devices

In the present embodiment, compounds (2-6) and (9-7) were used as the host material, $Ir(ppy)_3$ as the light-emitting material, HATCN as the hole injection material, NPB and TCTA as the hole transport material, and B3PYMPM as the electron transport material, to make an electroluminescent device have a device structure of ITO/HATCN/NPB/TCTA/host material: $Ir(ppy)_3$(15%)/B3PYMPMALiF/Al.

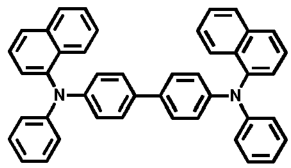

NPB

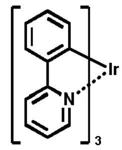

Ir(ppy)3

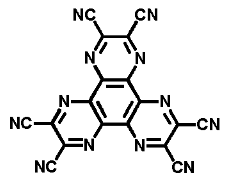

HATCN

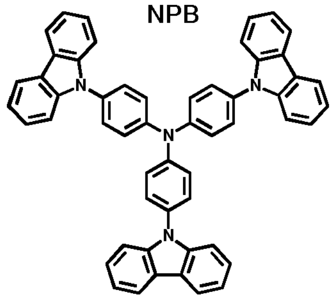

TCTA

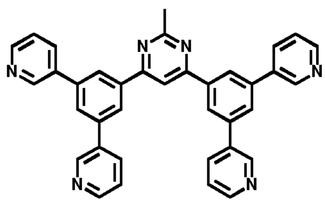

$B_3$PYMPM

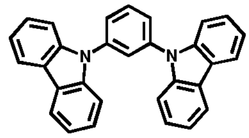

Ref1

The above materials such as HATCN, NPB, TCTA, B3PYMPM, $Ir(ppy)_3$ and Ref1 are all commercially available, such as from Jilin OLED Material Tech Co., Ltd ( www.jl-oled.com), or all the synthesis methods thereof are all known which can be found in the references of the art and will not be described here.

The preparation process of the above OLED device will be described in detail through a specific embodiment. The structure of the OLED device (as shown in Table 2) is: ITO/HATCN/NPB/TCTA/host material: $Ir(ppy)_3$/B3PYMPM/LiF/Al, and the preparation steps are as follows:

a. Cleaning of ITO (Indium Tin Oxide) conductive glass substrate: cleaning with a variety of solvents (such as one or more of chloroform, acetone or isopropanol), and then treating with ultraviolet and ozone;
b. Performing thermal evaporation in high vacuum ($1\times10^{-6}$ mbar), to form HATCN (5nm), NPB (40 nm), TCTA (10 nm), host material: 15% $Ir(ppy)_3$ (15 nm), B3PYMPM (40 nm), LiF (1 nm), Al (100 nm) );
c. Encapsulating: encapsulating the device with UV-curable resin in a nitrogen glove box.

Table 2: OLED devices

| OLED devices | Host materials |
|---|---|
| OLED1* | (2-6) |
| OLED2 | (9-7) |
| OLED3 | Ref1 |
| *Reference Example | |

The current-voltage (J-V) characteristics of each OLED device are characterized by characterization equipment while important parameters such as efficiency, lifetime, and external quantum efficiency are recorded. As detected, the light-emitting efficiency of OLED1 and OLED2 is more than 3 times that of RefOELD1, and the lifetime is 2 times that of RefOELD1. It can be seen that the lifetime of the OLED device prepared by using the organic compound of the present

disclosure have been greatly improved.

## Claims

1. A sulfone-containing fused heterocyclic compound with the following general formula (1):

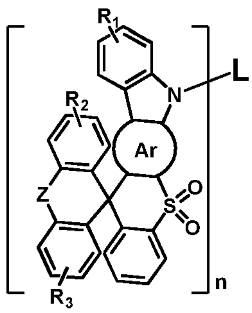

(1);

**characterized in that**, L represents an aromatic ring with a carbon atom number of 6 to 60, or a heteroaromatic ring with a carbon atom number of 3 to 60;

-Z- represents a single bond, -N(R)-, $-C(R)_2-$, $-Si(R)_2-$, -O-, -C=N(R)-, $-C=C(R)_2-$ , -P(R)-, -P(=O)R-, -S-, -(S=O)- or $-SO_2-$;
$R_1$, $R_2$, $R_3$ and R independently represents H, D, F, CN, aralkyl, alkenyl, alkynyl, nitrile, amine, nitro, acyl, alkoxy, carbonyl, sulfonyl, hydroxyl, alkyl with a carbon atom number of 1 to 30, cycloalkyl with a carbon atom number of 3 to 30, aromatic hydrocarbyl with a carbon atom number of 6 to 60, or aromatic heterocyclyl with a carbon atom number of 3 to 60 respectively;
Ar represents an aromatic ring with a carbon atom number of 6 to 40, or a heteroaromatic ring with a carbon atom number of 3 to 40;
n is selected from 2, 3, 4, 5, or 6.

2. The sulfone-containing fused heterocyclic compound according to claim 1, **characterized in that**, Ar in the general formula (1) is one structural group selected from the group consisting of:

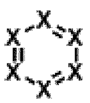 、 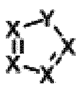 、 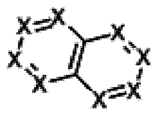 、 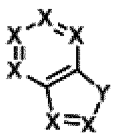 and 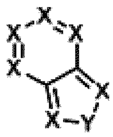 ;

wherein X is $CR^1$ or N;

Y is selected from the group consisting of $CR^2R^3$, $SiR^4R^5$, $NR^6$, C(=O), S and O;
$R^1$, $R^2$, $R^3$, $R^4$, $R^5$, and $R^6$ each independently represents H, or D, or linear alkyl groups containing 1 to 20 carbon atoms, linear alkoxy groups containing 1 to 20 carbon atoms or linear thioalkoxy groups containing 1 to 20 carbon atoms, or branched or cyclic alkyl groups containing 3 to 20 carbon atoms, branched or cyclic alkoxy groups containing 3 to 20 carbon atoms or branched or cyclic thioalkoxy groups containing 3 to 20 carbon atoms or branched or cyclic silyl groups containing 3 to 20 carbon atoms, or substituted keto groups containing 1 to 20 carbon atoms, alkoxycarbonyl groups containing 2 to 20 carbon atoms, aryloxycarbonyl groups containing 7 to 20 carbon atoms, cyano group, carbamoyl group , haloformyl group, formyl group, isocyano group, isocyanate group, thiocyanate group, or isothiocyanate group, hydroxyl group, nitro group, $CF_3$ group, Cl, Br, F, or substituted or unsubstituted aromatic or heteroaromatic ring systems containing 5 to 40 ring atoms, aryloxy or heteroaryloxy groups containing 5 to 40 ring atoms.

3. The sulfone-containing fused heterocyclic compound according to claim 1, **characterized in that** Ar in general formula (1) is selected from one of the following structural groups:

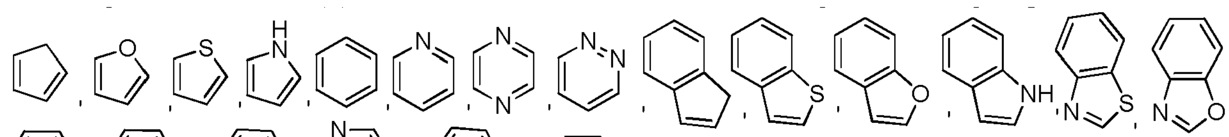

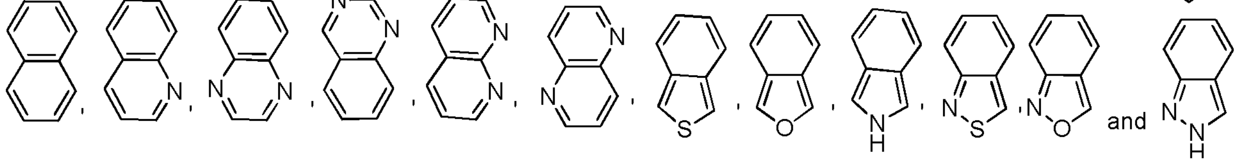

4. The sulfone-containing fused heterocyclic compound according to claim 1, **characterized in that** L in general formula (1) is selected from one of the following structural groups:

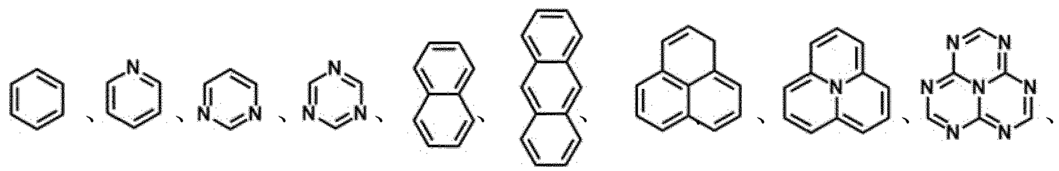

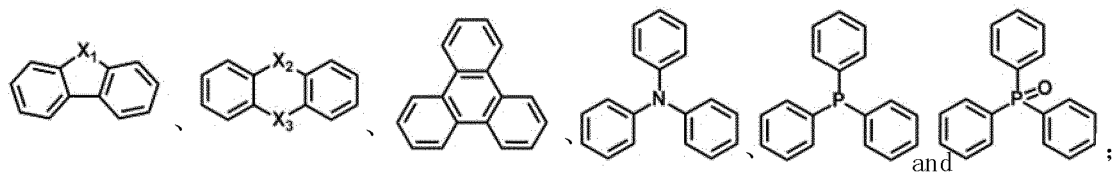

wherein $X_1$, $X_2$ and $X_3$ each independently represents N(R), $C(R)_2$, $Si(R)_2$, O, C=N(R), $C=C(R)_2$, P(R), P(=O)R, S, S=O, $SO_2$ or absent, wherein at least one of $X_2$ and $X_3$ is not absent; R represents H, D, F, CN, aralkyl, alkenyl, alkynyl, nitrile, amine, nitro, acyl, alkoxy, carbonyl, sulfonyl, hydroxyl, alkyl with a carbon atom number of 1 to 30, cycloalkyl with a carbon atom number of 3 to 30, aromatic hydrocarbyl with a carbon atom number of 6 to 60, or aromatic heterocyclyl with a carbon atom number of 3 to 60.

5. The sulfone-containing fused heterocyclic compound according to any one of claims 1 to 4, **characterized in that** the sulfone-containing fused heterocyclic compound is represented by any one of the following formulas (2) to (6):

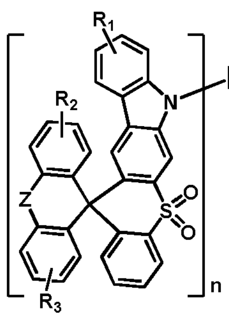 (2), 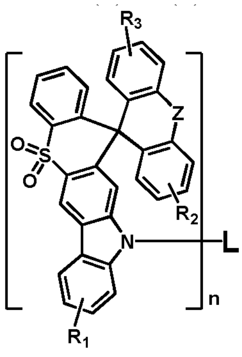 (3), 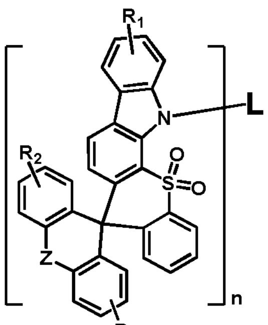 (4), 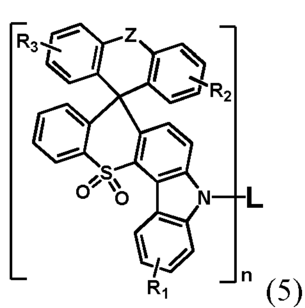 (5),

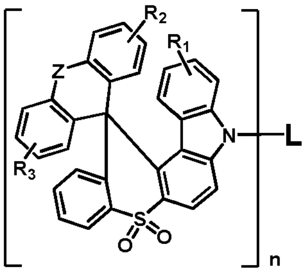

(6).

6. The sulfone-containing fused heterocyclic compound according to any one of claims 1 to 5, **characterized in that**, the sulfone-containing fused heterocyclic compound has a triplet energy level $T_1 \geq 2.2eV$.

7. The sulfone-containing fused heterocyclic compound according to any one of claims 1 to 6, **characterized in that**, the sulfone-containing fused heterocyclic compound has a difference between the singlet and triplet energy levels $\Delta(S_1-T_1) \leq 0.25eV$.

8. A polymer, **characterized in that**, the repeating unit of the polymer comprises one sulfone-containing fused heterocyclic structural unit according to any one of claims 1 to 7.

9. The polymer according to claim 8, **characterized in that**, the polymer is a non-conjugated polymer, wherein the sulfone-containing fused heterocyclic structural unit according to any one of claims 1 to 7 is located on the side chain of the polymer, or the polymer is a conjugated polymer, wherein the sulfone-containing fused heterocyclic structural unit according to any one of claims 1 to 7 is located on the backbone of the polymer.

10. A mixture, **characterized in that**, the mixture comprises one sulfone-containing fused heterocyclic compound according to any one of claims 1 to 7 and an organic functional material, or the mixture comprises one polymer according to any one of claims 8 to 9 and an organic functional material;
the organic functional material is at least one selected from the group consisting of a hole injection material, a hole transport material, an electron transport material, an electron injection material, an electron blocking material, a hole blocking material, an emitter, a host material, and an organic dye.

11. The mixture according to claim 10, **characterized in that**, the content of the sulfone-containing fused heterocyclic compound according to any one of claims 1 to 7 or the polymer according to any one of claims 8 to 9 in the mixture is 50 wt% to 99.9 wt%.

12. A formulation comprising at least one sulfone-containing fused heterocyclic compound according to any one of claims 1 to 7 and at least one organic solvent;

    alternatively, the formulation comprises at least one polymer according to any one of claims 8 to 9 and at least one organic solvent;
    alternatively, the formulation comprises at least one mixture according to any one of claims 10 to 11 and at least one organic solvent.

13. An organic electronic device comprising at least one sulfone-containing fused heterocyclic compound according to any one of claims 1 to 7, or at least one polymer according to any one of claims 8 to 9 or at least one mixture according to any one of claims 10 to 11.

14. The organic electronic device according to claim 13, **characterized in that**, the organic electronic device is at least one selected from of the group consisting of an organic light-emitting diode, an organic photovoltaic cell, an organic light-emitting electrochemical cell, an organic field effect transistor, an organic light-emitting field effect transistor, an organic sensor, and an organic plasmon emitting diode.

15. The organic electronic device according to claim 14, **characterized in that**, the organic electronic device is an electroluminescent device comprising a light-emitting layer which comprises at least one sulfone-containing fused heterocyclic compound according to any one of claims 1 to 7 and a light-emitting material, and

alternatively, the light-emitting layer comprises at least one polymer according to any one of claims 8 to 9 and a light-emitting material;
alternatively, the light-emitting layer comprises at least one mixture according to any one of claims 10 to 11 and a light-emitting material; the light-emitting material is selected from the group consisting of a fluorescent emitter, a phosphorescent emitter, a TADF material or a light-emitting quantum dot.

**Patentansprüche**

1. Sulfonhaltige kondensierte heterocyclische Verbindung mit der folgenden allgemeinen Formel (1):

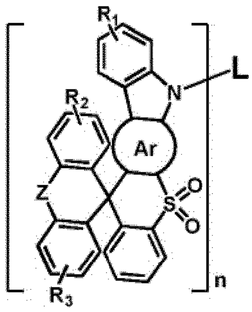 (1);

**dadurch gekennzeichnet, dass** L einen aromatischen Ring mit einer Kohlenstoffatomzahl von 6 bis 60 oder einen heteroaromatischen Ring mit einer Kohlenstoffatomzahl von 3 bis 60 darstellt;

-Z- eine Einfachbindung, -N(R)-, $-C(R)_2-$, $-Si(R)_2-$, -O-, -C=N(R)-, $-C=C(R)_2-$, -P(R)-, -P(=O)R-, -S-, -(S=O)- oder $-SO_2-$ darstellt;
$R_1$, $R_2$, $R_3$ und R unabhängig H, D, F, CN, Aralkyl, Alkenyl, Alkinyl, Nitril, Amin, Nitro, Acyl, Alkoxy, Carbonyl, Sulfonyl, Hydroxyl, Alkyl mit einer Kohlenstoffatomzahl von 1 bis 30, Cycloalkyl mit einer Kohlenstoffatomzahl von 3 bis 30, aromatisches Hydrocarbyl mit einer Kohlenstoffatomzahl von 6 bis 60 bzw. aromatisches Heterocyclyl mit einer Kohlenstoffatomzahl von 3 bis 60 darstellen;
Ar einen aromatischen Ring mit einer Kohlenstoffatomzahl von 6 bis 40 oder einen heteroaromatischen Ring mit einer Kohlenstoffatomzahl von 3 bis 40 darstellt;
n ausgewählt ist aus 2, 3, 4, 5 oder 6.

2. Sulfonhaltige kondensierte heterocyclische Verbindung gemäß Anspruch 1, **dadurch gekennzeichnet, dass** Ar in der allgemeinen Formel (1) eine strukturelle Gruppe ist, die ausgewählt ist aus der Gruppe bestehend aus:

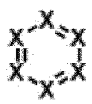 , 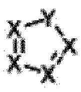 , 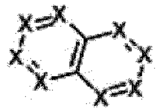 , 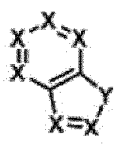 und 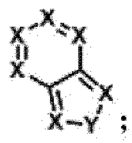 ;

worin X $CR^1$ oder N ist;

Y ausgewählt ist aus der Gruppe, bestehend aus $CR^2R^3$, $SiR^4R^5$, $NR^6$, C(=O), S und O;
$R^1$, $R^2$, $R^3$, $R^4$, $R^5$ und $R^6$ jeweils unabhängig H oder D, oder lineare Alkylgruppen mit 1 bis 20 Kohlenstoffatomen, lineare Alkoxygruppen mit 1 bis 20 Kohlenstoffatomen oder lineare Thioalkoxygruppen mit 1 bis 20 Kohlenstoffatomen, oder verzweigte oder cyclische Alkylgruppen mit 3 bis 20 Kohlenstoffatomen, verzweigte oder cyclische Alkoxygruppen mit 3 bis 20 Kohlenstoffatomen oder verzweigte oder cyclische Thioalkoxygruppen mit 3 bis 20 Kohlenstoffatomen oder verzweigte oder cyclische Silylgruppen mit 3 bis 20 Kohlenstoffatomen, oder substituierte Ketogruppen mit 1 bis 20 Kohlenstoffatomen, Alkoxycarbonylgruppen mit 2 bis 20 Kohlenstoffatomen, Aryloxycarbonylgruppen mit 7 bis 20 Kohlenstoffatomen, eine Cyanogruppe, eine Carbamoylgruppe, eine Haloformylgruppe, eine Formylgruppe, eine Isocyanogruppe, eine Isocyanatgruppe, eine Thiocyanatgruppe oder eine Isothiocyanatgruppe, eine Hydroxylgruppe, eine Nitrogruppe, eine $CF_3$-Gruppe, Cl, Br, F, oder substituierte oder unsubstituierte aromatische oder heteroaromatische Ringsysteme mit 5 bis 40 Ringatomen, Aryloxy- oder Heteroaryloxygruppen mit 5 bis 40 Ringatomen darstellen.

3. Sulfonhaltige kondensierte heterocyclische Verbindung gemäß Anspruch 1, **dadurch gekennzeichnet, dass** Ar in der allgemeinen Formel (1) ausgewählt ist aus einer der folgenden strukturellen Gruppen:

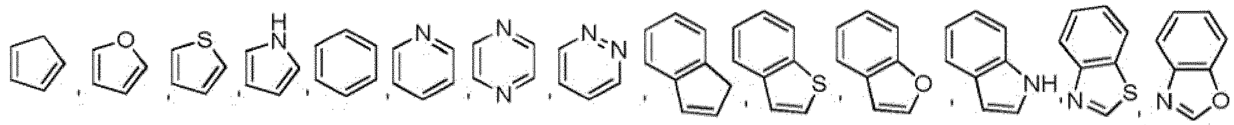

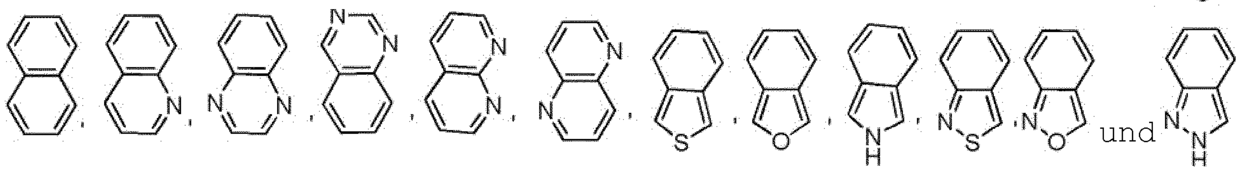

4. Sulfonhaltige kondensierte heterocyclische Verbindung gemäß Anspruch 1, **dadurch gekennzeichnet, dass** L in der allgemeinen Formel (1) ausgewählt ist aus einer der folgenden strukturellen Gruppen:

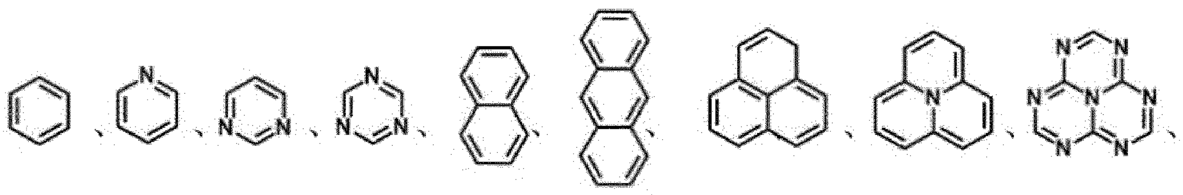

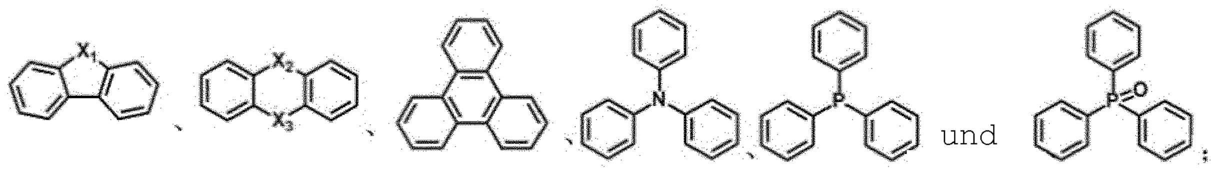

worin $X_1$, $X_2$ und $X_3$ jeweils unabhängig N(R), $C(R)_2$, $Si(R)_2$, O, C=N(R), $C=C(R)_2$, P(R), P(=O)R, S, S=O, $SO_2$ darstellen oder nicht vorhanden sind, wobei mindestens eines von $X_2$ und $X_3$ vorhanden ist; R H, D, F, CN, Aralkyl, Alkenyl, Alkinyl, Nitril, Amin, Nitro, Acyl, Alkoxy, Carbonyl, Sulfonyl, Hydroxyl, Alkyl mit einer Kohlenstoffatomzahl von 1 bis 30, Cycloalkyl mit einer Kohlenstoffatomzahl von 3 bis 30, aromatisches Hydrocarbyl mit einer Kohlenstoffatomzahl von 6 bis 60 oder aromatisches Heterocyclyl mit einer Kohlenstoffatomzahl von 3 bis 60 darstellt.

5. Sulfonhaltige kondensierte heterocyclische Verbindung gemäß mindestens einem der Ansprüche 1 bis 4, **dadurch gekennzeichnet, dass** die sulfonhaltige kondensierte heterocyclische Verbindung durch eine der folgenden Formeln (2) bis (6) dargestellt wird:

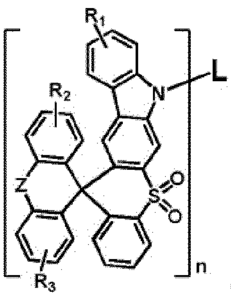 (2), 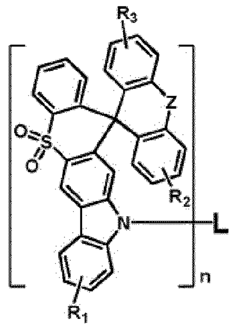 (3), 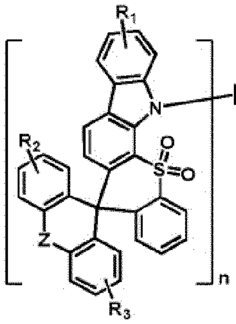 (4), 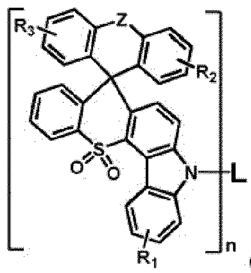 (5),

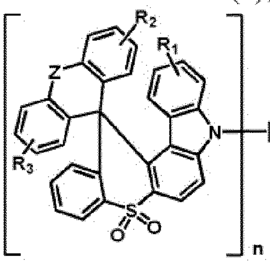 (6).

**6.** Sulfonhaltige kondensierte heterocyclische Verbindung gemäß mindestens einem der Ansprüche 1 bis 5, **dadurch gekennzeichnet, dass** die sulfonhaltige kondensierte heterocyclische Verbindung ein Triplett-Energieniveau $T_1 \geq$ 2,2eV aufweist.

**7.** Sulfonhaltige kondensierte heterocyclische Verbindung gemäß mindestens einem der Ansprüche 1 bis 6, **dadurch gekennzeichnet, dass** die sulfonhaltige kondensierte heterocyclische Verbindung eine Differenz zwischen den Singulett- und Triplett-Energieniveaus $\Delta(S_1\text{-}T_1) \leq$ 0,25eV aufweist.

**8.** Polymer, **dadurch gekennzeichnet, dass** die Wiederholeinheit des Polymers eine sulfonhaltige kondensierte heterocyclische Struktureinheit gemäß mindestens einem der Ansprüche 1 bis 7 umfasst.

**9.** Polymer gemäß Anspruch 8, **dadurch gekennzeichnet, dass** das Polymer ein nicht-konjugiertes Polymer ist, wobei die sulfonhaltige kondensierte heterocyclische Struktureinheit gemäß mindestens einem der Ansprüche 1 bis 7 an der Seitenkette des Polymers angeordnet ist, oder dass das Polymer ein konjugiertes Polymer ist, wobei die sulfonhaltige kondensierte heterocyclische Struktureinheit gemäß mindestens einem der Ansprüche 1 bis 7 an der Hauptkette des Polymers angeordnet ist.

**10.** Mischung, **dadurch gekennzeichnet, dass** die Mischung eine sulfonhaltige kondensierte heterocyclische Verbindung gemäß mindestens einem der Ansprüche 1 bis 7 und ein organisches funktionelles Material umfasst, oder dass die Mischung ein Polymer gemäß mindestens einem der Ansprüche 8 bis 9 und ein organisches funktionelles Material umfasst;
wobei das organische funktionelle Material mindestens eines ist, das ausgewählt ist aus einer Gruppe bestehend aus einem Lochinjektionsmaterial, einem Lochtransportmaterial, einem Elektronentransportmaterial, einem Elektroneninjektionsmaterial, einem Elektronensperrmaterial, einem Lochsperrmaterial, einem Emitter, einem Wirtsmaterial und einem organischen Farbstoff.

**11.** Mischung gemäß Anspruch 10, **dadurch gekennzeichnet, dass** der Gehalt der sulfonhaltigen kondensierten heterocyclischen Verbindung gemäß mindestens einem der Ansprüche 1 bis 7 oder des Polymers gemäß mindestens einem der Ansprüche 8 bis 9 in der Mischung 50 Gew.-% bis 99,9 Gew.-% beträgt.

**12.** Formulierung, umfassend mindestens eine sulfonhaltige kondensierte heterocyclische Verbindung gemäß mindestens einem der Ansprüche 1 bis 7 und mindestens ein organisches Lösungsmittel;

oder die Formulierung umfasst alternativ mindestens ein Polymer gemäß mindestens einem der Ansprüche 8 bis 9 und mindestens ein organisches Lösungsmittel;
oder die Formulierung umfasst alternativ mindestens eine Mischung gemäß mindestens einem der Ansprüche 10 bis 11 und mindestens ein organisches Lösungsmittel.

**13.** Organische elektronische Vorrichtung, umfassend mindestens eine sulfonhaltige kondensierte heterocyclische Verbindung gemäß mindestens einem der Ansprüche 1 bis 7 oder mindestens ein Polymer gemäß mindestens einem der Ansprüche 8 bis 9 oder mindestens eine Mischung gemäß mindestens einem der Ansprüche 10 bis 11.

**14.** Organische elektronische Vorrichtung gemäß Anspruch 13, **dadurch gekennzeichnet, dass** die organische elektronische Vorrichtung mindestens eine Vorrichtung ist, die ausgewählt ist aus einer Gruppe bestehend aus einer organischen Leuchtdiode, einer organischen Photovoltaikzelle, einer organischen lichtemittierenden elektrochemischen Zelle, einem organischen Feldeffekttransistor, einem organischen lichtemittierenden Feldeffekttransistor, einem organischen Sensor und einer organischen Plasmonen emittierenden Diode.

**15.** Organische elektronische Vorrichtung gemäß Anspruch 14, **dadurch gekennzeichnet, dass** die organische elektronische Vorrichtung eine Elektrolumineszenz-Vorrichtung ist, die eine lichtemittierende Schicht umfasst, wobei diese mindestens eine sulfonhaltige kondensierte heterocyclische Verbindung gemäß mindestens einem der Ansprüche 1 bis 7 und ein lichtemittierendes Material umfasst,

oder alternativ die lichtemittierende Schicht mindestens ein Polymer gemäß mindestens einem der Ansprüche 8 bis 9 und ein lichtemittierendes Material umfasst;
oder alternativ die lichtemittierende Schicht mindestens eine Mischung gemäß mindestens einem der Ansprüche 10 bis 11 und ein lichtemittierendes Material umfasst;
wobei das lichtemittierende Material ausgewählt ist aus einer Gruppe bestehend aus einem Fluoreszenzemitter, einem Phosphoreszenzemitter, einem TADF-Material oder einer Quantendotleuchtdiode.

## Revendications

**1.** Composé hétérocyclique fusionné contenant de la sulfone avec la formule générale suivante (1) :

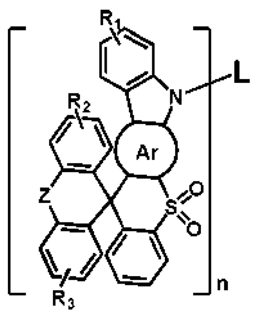

(1)

**caractérisé en ce que** L représente un cycle aromatique avec un nombre d'atomes de carbone de 6 à 60, ou un cycle hétéroaromatique avec un nombre d'atomes de carbone de 3 à 60 ;

-Z- représente une liaison unique, -N(R)-, $-C(R)_2-$, $-SI(R)_2-$, -O-, -C=N(R)-, $-C=C(R)_2-$, -P(R)-, -P(=O)R-, -S-, -(S=O)- or $-SO_2-$ ; $R_1$, $R_2$, $R_3$ et R représentent indépendamment H, D, F, CN, un aralkyle, alcényle, alcynyle, nitrile, amine, nitro, acyle, alcoxy, carbonyle, alkyle, hydroxyle, alkyle avec un nombre d'atomes de carbone de 1 à 30, un cycloalkyle avec un nombre d'atomes de carbone de 3 à 30, un hydrocarbyle aromatique avec un nombre d'atomes de carbone de 6 à 60, ou un hétérocyclyle aromatique avec un nombre d'atomes de carbone de 3 à 60, respectivement ;
AR représente un cycle aromatique avec un nombre d'atomes de carbone de 6 à 40, ou un cycle hétéroaromatique avec un nombre d'atomes de carbone de 3 à 40 ;
n est choisi parmi 2, 3, 4, 5 ou 6.

**2.** Composé hétérocyclique fusionné contenant de la sulfone selon la revendication 1, **caractérisé en ce que** Ar dans la formule générale (1) est un groupe structurel choisi dans le groupe constitué de :

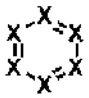、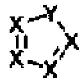、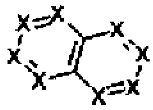、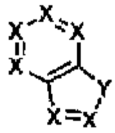 et 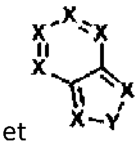 ;

dans lequel X est $CR^1$ ou N ;

Y est choisi dans le groupe consistant en $CR^2R^3$, $SiR^4R^5$, $NR^6$, C(=O), S et O ;
$R^1$, $R^2$, $R^3$, $R^4$, $R^5$ et $R^6$représentent chacun indépendamment H ou D, ou des groupes alkyle linéaires contenant de 1 à 20 atomes de carbone, des groupes alcoxy linéaires contenant de 1 à 20 atomes de carbone ou des groupes thioalcoxy linéaires contenant de 1 à 20 atomes de carbone , ou des groupes alkyle ramifiés ou cycliques contenant de 3 à 20 atomes de carbone, des groupes alcoxy ramifiés ou cycliques contenant de 3 à 20 atomes de carbone ou des groupes thioalcoxy ramifiés ou cycliques contenant de 3 à 20 atomes de carbone ou des groupes silyle ramifiés ou cycliques contenant de 3 à 20 atomes de carbone, ou des groupes céto substitués contenant de 1 à 20 atomes de carbone, des groupes alcoxycarbonyle contenant de 2 à 20 atomes de carbone, des groupes aryloxycarbonyle contenant de 7 à 20 atomes de carbone, un groupe cyano, un groupe carbamoyle, un groupe haloformyle, un groupe formyle, un groupe isocyano, un groupe isocyanate, un groupe thiocyanate, ou un groupe isothiocyanate, un groupe hydroxyle, un groupe nitro, un groupe $CF^3$, Cl, Br, F, ou des systèmes cycliques aromatiques ou hétéroaromatiques substitués ou non substitués contenant de 5 à 40 atomes cycliques, des groupes aryloxy ou hétéroaryloxy contenant de 5 à 40 atomes cycliques.

**3.** Composé hétérocyclique fusionné contenant de la sulfone selon la revendication 1, **caractérisé en ce que** Ar dans la formule générale (1) est choisi parmi l'un des groupes structuraux suivants :

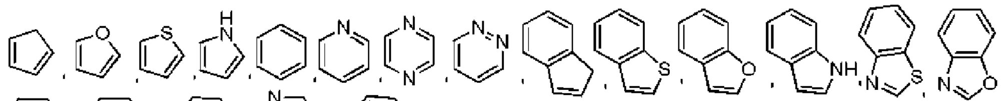

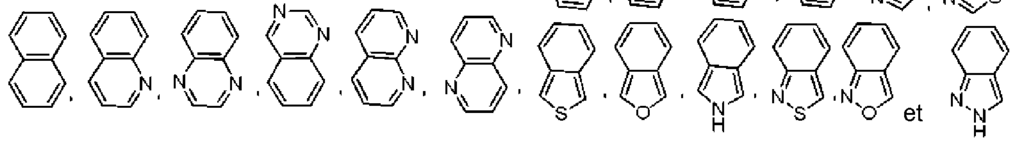

**4.** Composé hétérocyclique fusionné contenant de la sulfone selon la revendication 1, **caractérisé en ce que** L dans la formule générale (1) est choisi parmi un des groupes structuraux suivants :

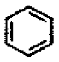、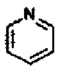、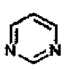、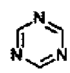、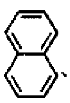、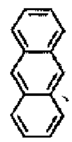、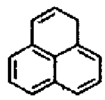、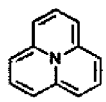、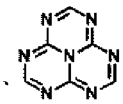、

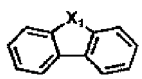、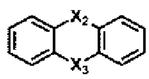、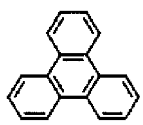、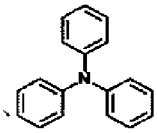、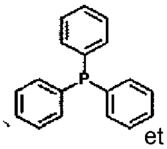 et 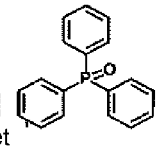 ;

dans lequel $X_1$, $X_2$ et $X_3$ représentent chacun indépendamment N(R), $C(R)_2$, $Si(R)_2$, O, C=N(R), $C=C(R)_2$, P(R), P(=O)R, S, S=O, $SO_2$ ou sont absents, où au moins un parmi $X_2$ et $X_3$ n'est pas absent ; R représente H, D, F, CN, un aralkyle, alcényle, alcynyle, nitrile, amine, nitro, acyle, alcoxy, carbonyle, sulfonyle, hydroxyle, alkyle avec un nombre d'atomes de carbone de 1 à 30, un cycloalkyle avec un nombre d'atomes de carbone de 3 à 30, un hydrocarbyle aromatique avec un nombre d'atomes de carbone de 6 à 60, ou un hétérocyclyle aromatique avec un nombre d'atomes de carbone de 3 à 60.

**5.** Composé hétérocyclique fusionné contenant de la sulfone selon l'une quelconque des revendications 1 à 4, **caractérisé en ce que** le composé hétérocyclique fusionné contenant de la sulfone est représenté par l'une quelconque des formules (2) à (6) suivantes :

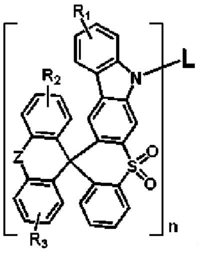 (2), 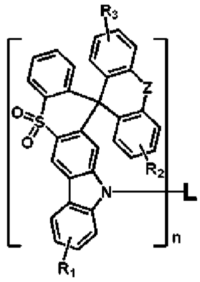 (3), 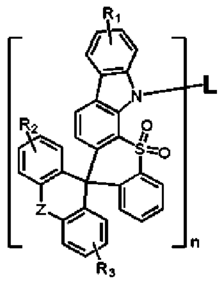 (4), 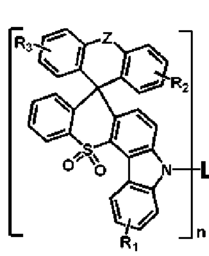 (5),

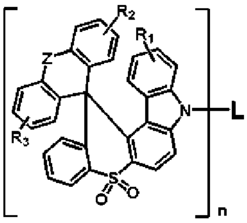 (6).

6. Composé hétérocyclique fusionné contenant de la sulfone selon l'une quelconque des revendications 1 à 5, **caractérisé en ce que** le composé hétérocyclique fusionné contenant de la sulfone présente un niveau d'énergie de triplet $T_1 \geq 2{,}2$ eV.

7. Composé hétérocyclique fusionné contenant de sulfone selon l'une quelconque des revendications 1 à 6, **caractérisé en ce que** le composé hétérocyclique fusionné contenant de la sulfone présente une différence entre les niveaux d'énergie de singulet et triplet $\Delta(S_1\text{-}T_1) \leq 0{,}25$ eV.

8. Polymère, **caractérisé en ce que** l'unité de répétition du polymère comprend une unité structurelle hétérocyclique fusionnée contenant de la sulfone selon l'une quelconque des revendications 1 à 7.

9. Polymère selon la revendication 8, **caractérisé en ce que** le polymère est un polymère non conjugué, dans lequel l'unité structurelle hétérocyclique fusionnée contenant de la sulfone selon l'une quelconque des revendications 1 à 7 est située sur la chaîne latérale du polymère, ou le polymère est un polymère conjugué, dans lequel l'unité structurelle hétérocyclique fusionnée contenant de la sulfone selon l'une quelconque des revendications 1 à 7 est située sur le squelette du polymère.

10. Mélange, **caractérisé en ce que** le mélange comprend un composé hétérocyclique fusionné contenant de la sulfone selon l'une quelconque des revendications 1 à 7 et un matériau fonctionnel organique, ou le mélange comprend un polymère selon l'une quelconque des revendications 8 à 9 et un matériau fonctionnel organique ;
le matériau fonctionnel organique est au moins un choisi dans le groupe constitué d'un matériau d'injection de trous, d'un matériau de transport de trous, d'un matériau de transport d'électrons, d'un matériau d'injection d'électrons, d'un matériau de blocage d'électrons, d'un matériau de blocage de trous, d'un émetteur, d'un matériau hôte, et d'un colorant organique.

11. Mélange selon la revendication 10, **caractérisé en ce que** la teneur du composé hétérocyclique fusionné contenant de la sulfone selon l'une quelconque des revendications 1 à 7 ou du polymère selon l'une quelconque des revendications 8 à 9 dans le mélange est de 50 % en poids à 99,9 % en poids.

12. Formulation comprenant au moins un composé hétérocyclique fusionné contenant de la sulfone selon l'une quelconque des revendications 1 à 7 et au moins un solvant organique ;

    en variante, la formulation comprend au moins un polymère selon l'une quelconque des revendications 8 à 9 et au moins un solvant organique ;
    en variante, la formulation comprend au moins un mélange selon l'une quelconque des revendications 10 à 11 et au moins un solvant organique.

13. Dispositif électronique organique comprenant au moins un composé hétérocyclique fusionné contenant de la sulfone selon l'une quelconque des revendications 1 à 7, ou au moins un polymère selon l'une quelconque des revendications 8 à 9 ou au moins un mélange selon l'une quelconque des revendications 10 à 11.

**14.** Dispositif électronique organique selon la revendication 13, **caractérisé en ce que** le dispositif électronique organique est au moins un choisi dans le groupe constitué d'une diode électroluminescente organique, d'une cellule photovoltaïque organique, d'une cellule électrochimique électroluminescente organique, d'un transistor à effet de champ, d'un transistor à effet de champ électroluminescent organique, d'un capteur organique et d'une diode émettrice organique à plasmons.

**15.** Dispositif électronique organique selon la revendication 14, **caractérisé en ce que** le dispositif électronique organique est un dispositif électroluminescent comprenant une couche électroluminescente qui comprend au moins un composé hétérocyclique fusionné contenant de la sulfone selon l'une quelconque des revendications 1 à 7 et un matériau électroluminescent, et

en variante, la couche électroluminescente comprend au moins un polymère selon l'une quelconque des revendications 8 à 9 et un matériau électroluminescent ;
en variante, la couche électroluminescente comprend au moins un mélange selon l'une quelconque des revendications 10 à 11 et un matériau électroluminescent; le matériau électroluminescent est choisi dans le groupe constitué par un émetteur fluorescent, un émetteur phosphorescent, un matériau TADF ou une boîte quantique électroluminescente.

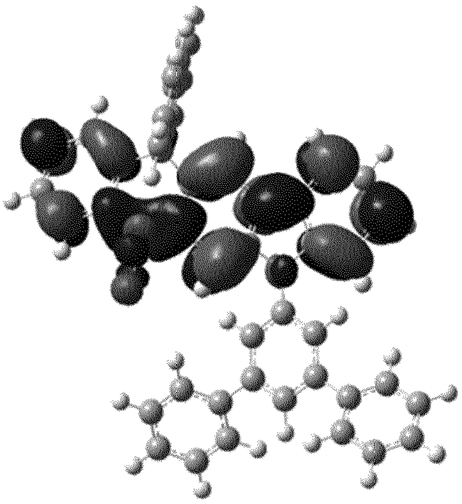

FIG. 1

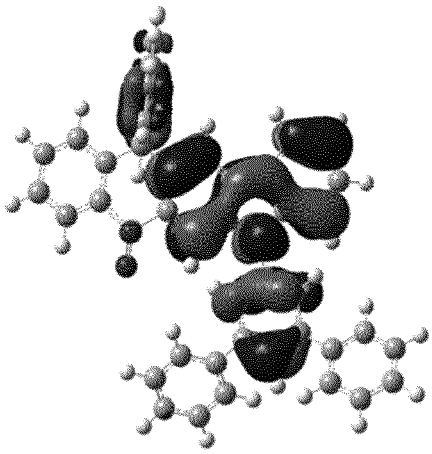

FIG. 2

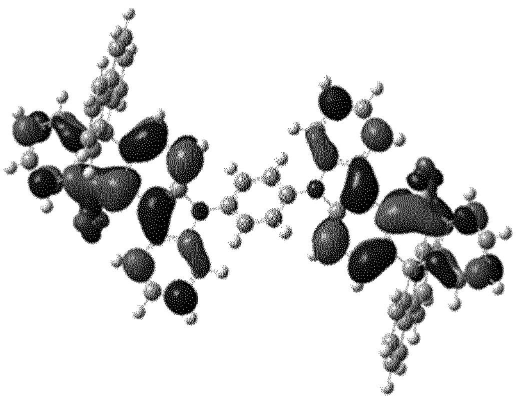

FIG. 3

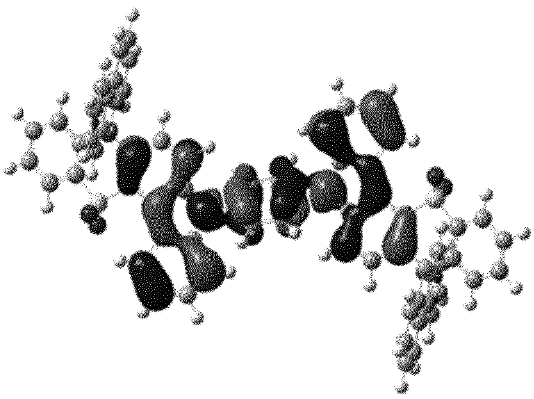

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 103718316
- KR 20110113469
- WO 2010135519 A1
- US 20090134784 A1
- WO 2011110277 A1
- JP 2913116 B
- WO 2001021729 A1
- WO 2008006449 A
- WO 2007140847 A
- WO 2006000388 A
- WO 2006058737 A
- WO 2006000389 A
- WO 2007065549 A
- WO 2007115610 A
- US 7250532 B2
- DE 102005058557 A1
- CN 1583691 A
- JP 08053397 A
- US 6251531 B1
- US 2006210830 A
- EP 1957606 A1
- US 20080113101 A1
- US 5121029 A
- WO 2006122630 A
- US 20060222886 A
- US 5130603 A
- US 20070092753 A1
- US 20070252517 A1
- US 4769292 A
- US 6020078 A
- CN 103483332 A
- TW 201309696 A
- TW 201309778 A
- TW 201343874 A
- TW 201350558 A
- US 20120217869 A1
- WO 2013133359 A1
- WO 2013154064 A1
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- US 20070087219 A1
- US 20090061681 A1
- US 20010053462 A1
- US 6824895 B
- US 7029766 B
- US 6835469 B
- US 6830828 B
- WO 2007095118 A1
- US 2012004407 A1
- WO 2012007088 A1
- WO 2012007087 A1
- WO 2012007086 A1
- US 2008027220 A1
- WO 2011157339 A1
- CN 102282150 A
- WO 2009118087 A1
- WO 2011141110 A

### Non-patent literature cited in the description

- **ADACHI et al.** *Nature,* 2012, vol. 492, 234
- Dendrimers and Dendrons. Wiley-VCH Verlag GmbH & Co. KGaA, 2002
- **ADACHI.** *Adv. Mater.,* 2009, vol. 21, 4802
- **ADACHI.** *Appl. Phys. Lett.,* 2011, vol. 98, 083302
- **ADACHI.** *Appl. Phys. Lett,* 2012, vol. 101, 093306
- **ADACHI.** *Chem. Commun.,* 2012, vol. 48, 11392
- **ADACHI.** *Nature Photonics,* 2012, vol. 6, 253
- **ADACHI.** *Nature,* 2012, vol. 492, 234
- **ADACHI.** *J. Am. Chem. Soc,* 2012, vol. 134, 14706
- **ADACHI.** *Angew. Chem. Int. Ed,* 2012, vol. 51, 11311

- **ADACHI.** *Chem. Commun.,* 2012, vol. 48, 9580
- **ADACHI.** *Chem. Commun.,* 2013, vol. 48, 10385
- **ADACHI.** *Adv. Mater.,* 2013, vol. 25, 3319
- **ADACHI.** *Adv. Mater.,* 2013, vol. 25, 3707
- **ADACHI.** *Chem. Mater.,* 2013, vol. 25, 3038
- **ADACHI.** *Chem. Mater.,* 2013, vol. 25, 3766
- **ADACHI.** *J. Mater. Chem. C.,* 2013, vol. 1, 4599
- **ADACHI.** *J. Phys. Chem. A.,* 2013, vol. 117, 5607
- **BALDO, THOMPSON et al.** *Nature,* 2000, vol. 403, 750-753
- **ADACHI et al.** *Appl. Phys. Lett.,* 2001, vol. 78, 1622-1624
- **J. KIDO et al.** *Appl. Phys. Lett.,* 1994, vol. 65, 2124
- **KIDO et al.** *Chem. Lett.,* 1990, vol. 657
- **JOHNSON et al.** *JACS,* 1983, vol. 105, 1795
- **WRIGHTON.** *JACS,* 1974, vol. 96, 998
- **MA et al.** *Synth. Metals,* 1998, vol. 94, 245
- **BULOVIC et al.** *Nature,* 1996, vol. 380, 29
- **GU et al.** *Appl. Phys. Lett.,* 1996, vol. 68, 2606